# FASCICULE DE BREVET EUROPEEN

(11) **EP 0 591 046 B1**
(45) Date de publication et mention de la délivrance du brevet: **02.12.1998**
(21) Numéro de dépôt: 93402372.2
(22) Date de dépôt: 29.09.1993
(51) Int. Cl.: C07D 311/58, C07D 311/64, C07D 311/70, C07F 9/655, A61K 31/35, A61K 31/665

(54) **Dérivés chromeniques à chaîne triénique, utilisables dans le traitement de l'ostéoporose et des affections inflammatoires**
Chromen-Derivate mit einer Trien-Kette zur Verwendung bei der Behandlung der Osteoporose und der entzündlichen Erkrankungen
Chromene derivatives with a triene chain for use in the treatment of osteoporosis inflammatory diseases

(30) Priorité: 29.09.1992 FR 9211592; 29.09.1992 FR 9211591
(43) Date de publication de la demande: 06.04.1994
(73) Titulaire: ADIR ET COMPAGNIE, 92415 Courbevoie Cédex (FR)
(72) Inventeur: Brion, Jean-Daniel, F-95320 Saint-Leu-la-Foret (FR); Le Baut, Guillaume, F-44230 Saint-Sebastien-sur-Loire (FR); Poissonnet, Guillaume, F-Villebon s/Yvette (FR); De Montarby, Lucy, F-92400 Courbevoie Cedex (FR); Belachmi, Larbi, F-44000 Nantes (FR); Bonnet, Jacqueline, F-75013 Paris (FR); Sabatini, Massimo, F-92380 Garges (FR); Tordjman, Charles, F-92100 Boulogne (FR)

(56) Documents cités:
- EP-A- 0 337 885
- GB-A- 2 188 634

## Description

La présente invention concerne de nouveaux dérivés chroméniques à chaîne latérale triénique, leur procédé de préparation, les compositions pharmaceutiques les renfermant ainsi que leur application dans le traitement de l'ostéoporose et des affections inflammatoires.

Il est connu que l'ostéoporose peut être traitée en inhibant la résorption osseuse ou en favorisant la formation du tissu osseux.

Les composés utilisés dans le traitement de l'ostéoporose sont soit des agents anti-résorbants, comme la calcitonine et ses dérivés, soit des agents générateurs de tissu osseux, comme les sels de fluor.

Les composés de l'invention possèdent une structure originale constituée d'une chaîne triénique fixée sur une structure bicyclique de type chromène éventuellement substituée. Ces composés présentent un intérêt pharmacologique supérieur aux composés de référence par le fait qu'ils agissent à la fois comme anti-résorbants osseux et comme régénérateurs du tissu osseux et ce, de manière plus rapide et à dose plus faible.

De plus, les composés de l'invention sont doués d'un pouvoir inhibiteur de la phospholipase A₂ entrant en jeu dans tout processus d'inflammation. On sait que le processus inflammatoire peut induire une résorption au niveau de l'os, l'ostéopénie.

Ces trois propriétés montrent que les composés de l'invention présentent un moyen de traitement complet et unique des principales maladies osseuses par leur pouvoir anti-résorbant osseux, générateur de tissu ostéoïde et anti-ostéopéniant.

Plus précisément, la présente invention a pour objet les composés de formule générale (I)**:** 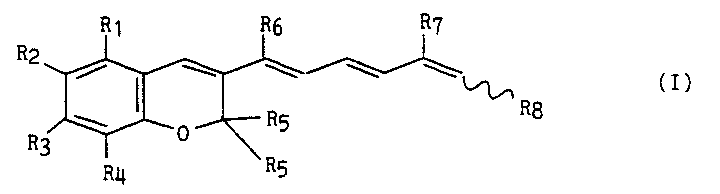 dans laquelle :
- R₁, R₂, R₃, R₄, identiques ou différents, sont choisis parmi :
   - un atome d'hydrogène,
   - un atome d'halogène,
   - un radical phényle substitué ou non,
   - un radical adamantyle,
   - un radical hétérocyclique substitué ou non, contenant 5 ou 6 atomes, choisi parmi les radicaux furyle, pyrrolyle, pyrrolidinyle, thiényle, pyridyle, pipéridyle, indolyle et quinolyle,
   - un radical R' et
   - un radical OR',
      - R' étant un groupement alkyle, linéaire ou ramifié contenant de 1 à 6 atomes de carbone, éventuellement substitué par un ou plusieurs groupements hydroxy, et/ou par un ou plusieurs atomes d'halogènes,
- R₅, R₆ et R₇, identiques ou différents, sont choisis parmi un atome d'hydrogène et un groupement alkyle linéaire ou ramifié contenant de 1 à 6 atomes de carbone,
- R₈ est choisi parmi les groupements C(O)OR₉, P(O)(OR₉)₂ et CONHR₁₀ dans lesquels R₉ est un atome d'hydrogène, un groupement alkyle linéaire ou ramifié contenant de 1 à 6 atomes de carbone ou un résidu d'un amino-sucre, par exemple le glucuronide, et R₁₀ représente un atome d'hydrogène, un groupement alkyle linéaire ou ramifié contenant de 1 à 6 atomes de carbone ou un radical aryle éventuellement substitué,
   la liaison indéfinie portant R₈ étant une liaison simple conférant une configuration E ou Z à la double liaison qui la supporte,
   avec la restriction que lorsque R₅ représente l'hydrogène et R₆ et R₇ représentent chacun le groupement méthyle, et R₈ représente le groupement C(O)OR₉ tel que précédemment défini, alors au moins un des substituants R₁, R₂, R₃ ou R₄ est différent de l'hydrogène, d'un halogène, d'un radical R', et d'un radical OR', R' étant tel que défini précédemment,
   leurs stéréoisomères ainsi que leurs éventuels sels d'addition à un acide ou à une base, pharmaceutiquement acceptables.

La présente invention s'étend également au procédé de préparation des composés de formule (I), caractérisé en ce que le composé de formule (II) : 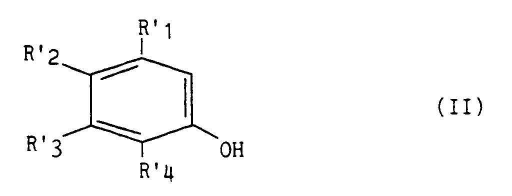 dans laquelle R'₁, R'₂, R'₃ et R'₄, identiques ou différents, représentent un atome d'hydrogène ou d'halogène, un groupement alkyle ou alkoxy linéaire ou ramifié contenant de 1 à 6 atomes de carbone, éventuellement substitués par un ou plusieurs groupements OH, et/ou par un ou plusieurs atomes d'halogène,
est transformé en composé de formule (III) : 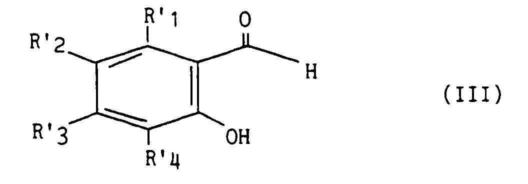 dans laquelle R'₁, R'₂, R'₃ et R'₄ ont les mêmes significations que précédemment,
selon la réaction de Sommelet, par action du para-formaldéhyde en présence d'hexaméthylènetétramine, à une température de 100°C, puis acidification, ou bien par action d'un chlorure métallique, par exemple le tétrachloroétain, en présence d'une alkylamine, comme la trioctylamine, avec le trioxyméthylène, également à 100°C,
composé de formule (III) qui est ensuite cyclisé par action d'un composé de formule (IV) : 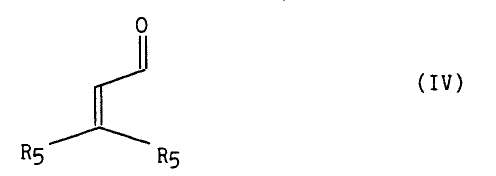 dans laquelle R₅ est tel que défini dans la formule (I),
en présence d'un carbonate alcalin, tel que le carbonate de potassium, dans un solvant polaire à reflux, tel que le dioxane ou la butan-2-one, pour conduire au composé de formule (V) : 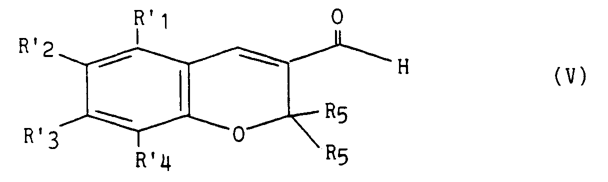 dans laquelle R'₁, R'₂, R'₃, R'₄ et R₅ sont tels que définis précédemment,
composé de formule (V) dont les groupements R'₁, R'₂, R'₃ et R'₄ lorsque l'un ou plusieurs d'entre eux représentent un atome d'halogène peuvent être substitués par un radical phényle, phényle substitué, adamantyle, ou un radical hétérocyclique tel que défini précédemment, par réaction avec un tétra-phényl-, -aryl-, -hétérocyclyl-, ou -hétéroaryl-étain correspondant, en présence d'un catalyseur à base de palladium, comme par exemple le trans-benzyl(chloro)bis(triphénylphosphine)palladium (II) dans le toluène à reflux, de façon à obtenir l'ensemble des composés de formule (VI) : 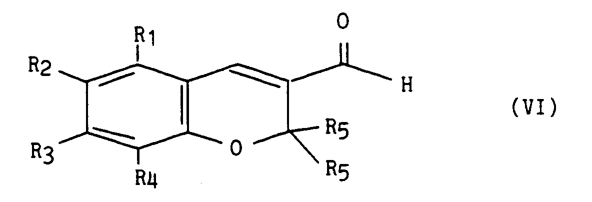 dans laquelle R₁, R₂, R₃, R₄ et R₅ ont les mêmes significations que précédemment,
qui, par réaction de Wittig à l'aide d'un composé de formule (VII) : 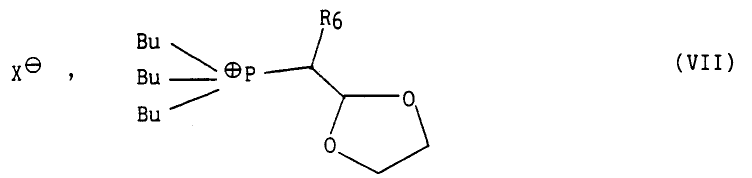 où X est un atome d'halogène et R₆ est tel que défini précédemment,
dans un solvant tel que la diméthylformamide puis hydrolyse acide, conduisent au composé de formule (VIII) : 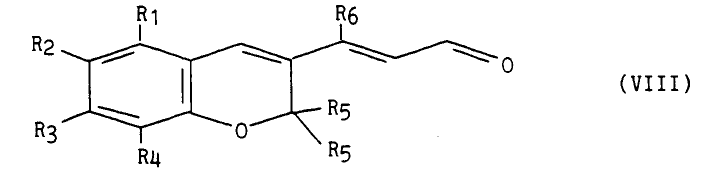 où R₁, R₂, R₃, R₄, R₅ et R₆ sont tels que définis précédemment,
composé de formule (VIII) qui, après une seconde réaction de Wittig, dans les mêmes conditions, avec un composé de formule (VII') : 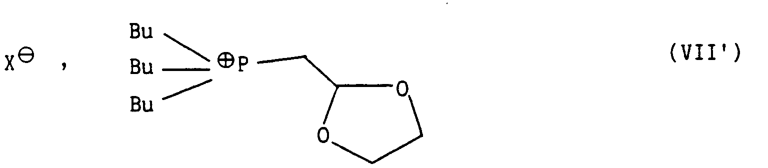 dans laquelle X est tel que défini précédemment,
est transformé en composé de formule (IX) : 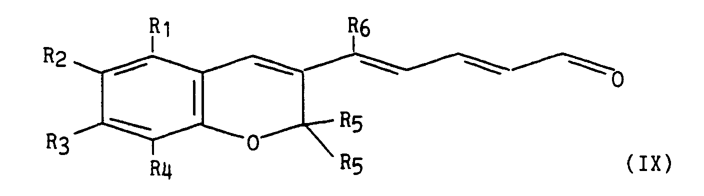 dans laquelle les groupements R₁ à R₆ sont tels que définis précédemment,
qui est finalement traité par un composé de formule (Xa) ou (Xb) : 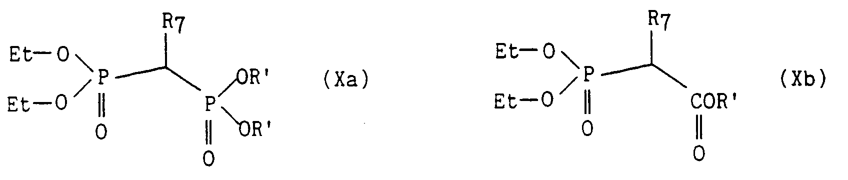 dans laquelle R₇ et R' sont tels que définis précédemment,
pour conduire respectivement aux composés de formules (Ia) et (Ib) : 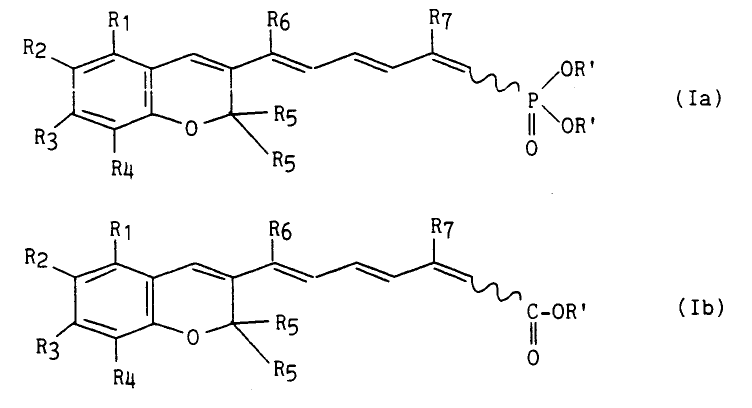 dans lesquelles les groupements R₁ à R₇ et R' sont tels que définis précédemment,
composé de formule (Ia) qui, traité en milieu basique ou acide, permet d'obtenir le composé de formule (Ic) : 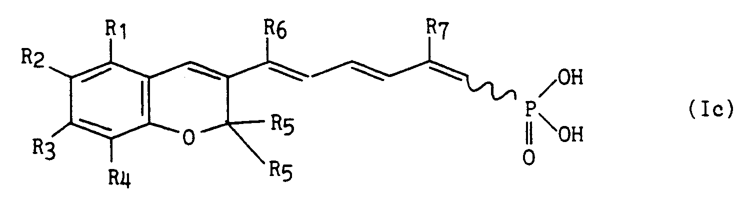 dans laquelle les groupements R₁ à R₇ sont tels que définis précédemment,
composé de formules (Ib) qui peut être saponifié en composé de formule (Id) : 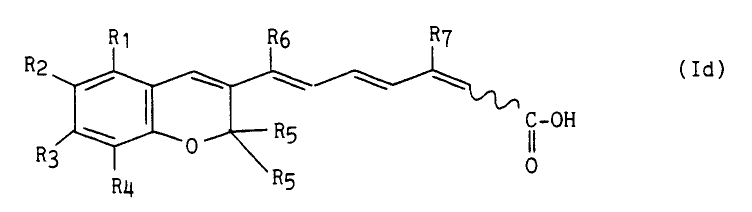 dans laquelle les groupements R₁ à R₇ sont tels que définis précédemment,
qui, à son tour, après transformation en son chlorure d'acyle correspondant, et traitement par une amine de formule (XI) :

R₁₀-NH₂ (XI)

dans laquelle R₁₀ est tel que défini précédemment,
conduit au composé de formule (Ie) : 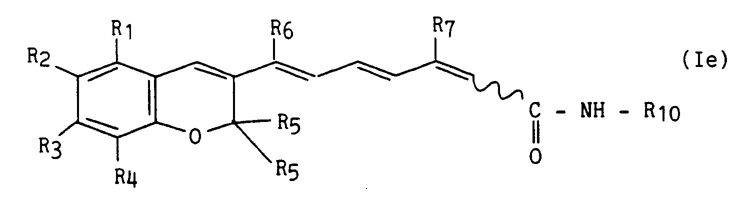 dans laquelle les groupements R₁ à R₇ et R₁₀ sont tels que définis précédemment,
l'ensemble des composés de formule (Ia) à (Ie) formant l'ensemble des composés de formule générale (I) que l'on purifie le cas échéant selon une technique classique de purification, dont on sépare, si on le souhaite,
les stéréoisomères par une technique classique de séparation et que l'on transforme si on le souhaite, en leurs sels d'addition à un acide ou à une base, pharmaceutiquement acceptables.

Les nouveaux composés de la présente invention possèdent des propriétés pharmacologiques et thérapeutiques intéressantes. En particulier pour ces dérivés, ont été mises en évidence leurs propriétés anti-ostéoporotiques, leur capacité de formation de tissu osseux ainsi que leur activité anti-inflammatoire en général.

D'une façon générale, les dérivés de la présente invention trouvent leur utilisation dans le traitement préventif ou non de l'ostéoporose par leur propriété antirésorbante, leur capacité à former le tissu osseux et leur action anti-inflammatoire permettant le traitement des affections impliquant la PLA₂, et en particulier le traitement des inflammations chroniques ostéopéniantes généralisées comme la polyarthrite rhumatoïdale, ou localisées, comme la parodontite.

La présente invention s'étend également à l'utilisation des composés de formule (I') : 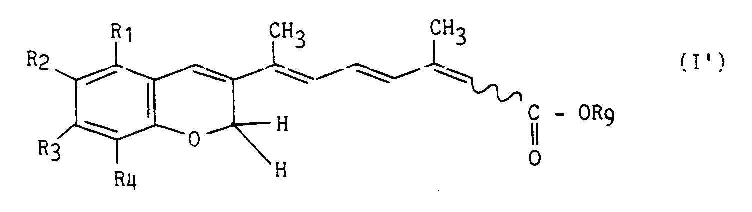 dans laquelle :
- R₁, R₂, R₃ et R₄, identiques ou différents sont choisis parmi :
   - un atomme d'hydrogène,
   - un atome d'halogène,
   - un radical R' et
   - un radical OR',
      - R' étant un groupement alkyle, linéaire ou ramifié, contenant de 1 à 6 atomes de carbone et éventuellement substitués par un ou plusieurs groupements hydroxy, et/ou par un ou plusieurs atomes d'halogène,
   - R₉ est choisi parmi un atome d'hydrogène, un groupement alkyle linéaire ou ramifié contenant de 1 à 6 atomes de carbone ou un résidu d'un amino-sucre, par exemple le glucuronide,
      la liaison indéfinie portant le radical 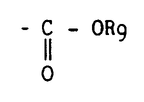 étant une liaison simple, conférant une configuration Z ou E à la double liaison qui la supporte,
      ainsi qu'à leurs éventuels stéréoisomères et sels d'addition à un acide ou à une base, pharmaceutiquement acceptables, (composés décrits dans le brevet EP-A-337885) pour l'obtention de compositions pharmaceutiques utiles dans le traitement de l'ostéoporose et des affections inflammatoires et en particulier dans les inflammations chroniques ostéopéniantes généralisées, comme la polyarthrite rhumatoïde, ou localisée, comme la parodontite.

L'invention s'étend aussi aux compositions pharmaceutiques renfermant comme principe actif au moins un composé de formule générale (I) ou (I') ou un de ses sels d'addition à un acide ou à une base pharmaceutiquement acceptable, seul ou en combinaison avec un ou plusieurs excipients inertes, non toxiques et appropriés.

Les compositions pharmaceutiques ainsi obtenues se présenteront sous diverses formes, les plus avantageuses étant les gélules, suppositoires, solutions injectables ou buvables, patches, les comprimés simples, dragéifiés ou sublinguaux, les sachets, paquets, glossettes, tablettes, crèmes, pommades, gels dermiques, aérosols.

La posologie utile est adaptable selon la nature et la sévérité de l'affection, la voie d'administration ainsi que selon l'âge et le poids du patient. De manière générale, la posologie unitaire s'échelonnera entre 10 mg à 200 mg par jour en une ou deux prises.

Les exemples suivants illustrent l'invention sans la limiter d'aucune façon. Les produits de départ sont connus ou préparés à partir de modes opératoires connus. Les exemples 32 à 38 et 40 à 45 appartiennent au brevet EP-A-337885.

### Exemple 1 : Acide (2E,4E,6E)-7-(6,8-diméthyl-2H-chromén-3-yl)hepta-2,4,6-triénoïque

### Etape A : 3,5-Diméthylsalicylaldéhyde

Un mélange de 30,5 g (250 mmoles) de 2,4-diméthylphénol, 18,9 g (135 mmoles) d'hexaméthylènetétramine et de 18,9 g (210 mmoles) de paraformaldéhyde est chauffé à 100°C sous agitation. 75 ml d'acide acétique sont ensuite ajoutés goutte à goutte. L'ensemble, sous agitation, est porté à 110°C pendant 15 minutes. 17 ml d'acide sulfurique et 6 ml d'eau sont additionnés pendant 15 minutes goutte à goutte. L'agitation est maintenue à 110°C pendant 30 minutes, puis le mélange est versé sur 500 ml d'eau chaude. L'ensemble est extrait à 3 reprises par le dichlorométhane, puis les phases organiques réunies sont lavées jusqu'à neutralité par une solution aqueuse d'hydrogénocarbonate de sodium. Après séchage sur sulfate de sodium, évaporation et distillation du solvant, 30,04 g d'une huile jaune sont recueillis.
- Rendement :: 80 %
- Spectre IR :: (KBr) ν_{OH} = 3200 à 2800 cm⁻¹
ν_{CH₃} = 2970 cm⁻¹ ; 2910 cm⁻¹
ν_{OC=O} = 1655 cm⁻¹

### Etape B : (6,8-Diméthyl-2H-chromén-3-yl)carboxaldéhyde

A une solution de 11,48 g (83,22 mmoles) de carbonate de potassium dans 120 ml de dioxane anhydre, sont ajoutés, à température ambiante, 7,9 g (52,6 mmoles) du composé obtenu à l'étape A ainsi que 4,66 g (83,22 mmoles) d'acroléine. Le mélange est porté à reflux sous agitation pendant 2 heures. Le milieu réactionnel est évaporé au bain-marie, sous vide, le résidu repris par 200 ml d'eau, et extrait à trois reprises par 100 ml d'éther diéthylique. Les phases organiques réunies sont lavées jusqu'à neutralité par une solution saturée aqueuse de chlorure de sodium, puis séchées sur sulfate de sodium. Après filtration, concentration et purification sur gel de silice (éluant : dichlorométhane/éther de pétrole : 50/50) du résidu huileux, 7,82 g de produit sont obtenus.
Rendement : 52 %
Point de fusion : 52-54°C

### Etape C : (E)-2-(6,8-Diméthyl-2H-chromén-3-yl)-1-(1,3-dioxolan-2-yl)éthène

6,67 g (18,7 mmoles) de bromure de tributyl-(1,3-dioxolan-2-yl)méthylphosphonium (obtenu par réaction de tributylphosphine sur le 2-bromoéthyl-1,3-dioxolane) et 3,52 g (18,7 mmoles) du composé obtenu à l'étape B sont mélangés à 120 ml de diméthylformamide anhydre. L'ensemble est chauffé à 90°C, puis sous atmosphère d'azote, 1,27 g (18,7 mmoles) d'éthylate de sodium (solution 1M dans l'éthanol absolu) sont ajoutés goutte à goutte. Après 16 heures à 90°C, le diméthylformamide est évaporé sous pression réduite et le milieu réactionnel repris par 150 ml d'eau. Après traitement habituel de la phase organique, le résidu est purifié par élution au dichlorométhane sur gel de silice pour conduire après évaporation du solvant à 2,66 g de l'acétal attendu.
Rendement : 55 %
Point de fusion : 62-64°C

### Etape D : (2E)-3-(6,8-Diméthyl-2H-chromén-3-yl)propénal

2,6 g (10,05 mmoles) de l'acétal obtenu à l'étape C sont solubilisés dans 80 ml de tétrahydrofurane, auxquels sont ajoutés 80 ml d'une solution aqueuse d'acide chlorhydrique 2,5 M.

La réaction se poursuit 2 heures à température ambiante puis le milieu réactionnel est hydrolysé par 250 ml d'eau. Après reprise par l'éther éthylique, traitement habituel de la phase organique et purification par chromatographie sur colonne de silice (éluant : éther de pétrole/dichlorométhane : 50/50), 2,15 g de cristaux jaunes recristallisés dans l'éther de pétrole sont obtenus.
Rendement : 85 %
Point de fusion : 76-78°C

### Etape E : (2E,4E)-5-(6,8-Diméthyl-2H-chromén-3-yl)-penta-2,4-diénal

3,31 g (15,47 mmoles) du propénal obtenu à l'étape D sont de nouveau soumis à la réaction de Wittig décrite à l'étape C ; l'acétal ainsi obtenu est hydrolysé selon la méthode décrite dans l'étape D. 2,53 g de cristaux oranges recristallisés dans l'éther isopropylique sont obtenus.
Rendement : 68 %
Point de fusion : 109-111°C

### Etape F : (2E,4E,6E)-7-(6,8-Diméthyl-2H-chromén-3-yl)hepta-2,4,6-triénoate d'éthyle

A un mélange de 0,083 g (2,16 mmoles) d'hydrure de sodium et de 80 ml de benzène anhydre refroidi à -5°C sont ajoutés goutte à goutte 0,43 ml (2,16 mmoles) de diéthylphosphonoacétate d'éthyle. L'ensemble est abandonné pendant 45 mn jusqu'au retour à température ambiante, puis refroidi à nouveau à -5°C. Une solution benzénique de 0,52 g (2,16 mmoles) de chroménylpentadiénal obtenu à l'étape E est alors ajoutée goutte à goutte ; après 4 heures à température ambiante, le solvant est évaporé sous vide, le milieu réactionnel est repris par 150 ml d'eau, puis par le dichlorométhane. Après traitement habituel de la phase organique et purification sur colonne de silice (éluant : dichlorométhane/éther de pétrole : 75/25), 0,54 g d'une poudre jaune sont obtenus, correspondant à l'isomère E.
Rendement : 70 %
Point de fusion : 108-110°C

### Etape G : Acide (2E,4E,6E)-7-(6,8-diméthyl-2H-chromén-3-yl)hepta-2,4,6-triénoïque

0,561 g (1,41 mmoles) d'ester obtenu à l'étape F et 1,15 ml (5,66 mmoles) d'une solution aqueuse d'hydroxyde de sodium 5N sont chauffés à 90°C pendant 4 heures dans 80 ml d'éthanol. Après évaporation du solvant et reprise du milieu réactionnel par 40 ml d'eau, celui-ci est acidifié par une solution d'acide chlorhydrique 1N. Le précipité formé est filtré, lavé et recristallisé dans l'éthanol pour donner 0,34 g d'une poudre jaune.
Rendement : 85 %
Point de fusion : 208-210°C

### Exemple 2 : Acide (2Z,4E,6E)-7-(6,8-diméthyl-2H-chromén-3-yl)hepta-2,4,6-triénoïque

Composé obtenu selon le même schéma que l'exemple 1 en isolant, lors de la purification décrite dans l'étape F, l'isomère 2Z.

Les exemples suivants sont obtenus de manière analogue à l'exemple 1.

### Exemple 3 : Acide (2E,4E,6E)-7-(2H-chromén-3-yl)hepta-2,4,6-triénoïque

Point de fusion : 206-208°C
   (cristaux jaunes, solvant : THF)

### Exemple 4 : Acide (2E,4E,6E)-7-(6-méthyl-2H-chromén-3-yl)hepta-2,4,6-triénoïque

Point de fusion : 235-240°C
   (cristaux jaunes, solvant : THF/éther de pétrole, 80:20)

### Exemple 5 : Acide (2E,4E,6E)-7-(6-isopropyl-2H-chromén-3-yl)hepta-2,4,6-triénoïque

Point de fusion : 244-246°C
   (cristaux jaunes, solvant : THF)

### Exemple 6 : Acide (2E,4E,6E)-7-(6-tert-butyl-2H-chromén-3-yl)hepta-2,4,6-triénoïque

Point de fusion : 200-202°C
   (cristaux oranges, solvant : THF)

### Exemple 7 : Acide (2E,4E,6E)-7-(6-trifluorométhyl-2H-chromén-3-yl)hepta-2,4,6-triénoïque

Point de fusion : 208-210°C
   (cristaux oranges, solvant : THF/eau, 80:20)

### Exemple 8 : Acide (2E,4E,6E)-7-(6-méthoxy-2H-chromén-3-yl)hepta-2,4,6-triénoïque

Point de fusion : 250-252°C
   (cristaux jaunes, solvant : THF)

### Exemple 9 : Acide (2E,4E,6E)-7-(7-méthoxy-2H-chromén-3-yl)hepta-2,4,6-triénoïque

Point de fusion : 238-240°C
   (cristaux jaunes, solvant : THF)

### Exemple 10 : Acide (2E,4E,6E)-7-(8-méthoxy-2H-chromén-3-yl)hepta-2,4,6-triénoïque

Point de fusion : 226-228°C
   (cristaux jaunes, solvant : THF)

### Exemple 11 : Acide (2E,4E,6E)-7-(6-méthyl-8-tert-butyl-2H-chromén-3-yl) hepta-2,4,6-triénoïque

Point de fusion : 256-258°C
   (cristaux jaunes, solvant : THF/éthanol, 80:20)

### Exemple 12 : Acide (2E,4E,6E)-7-(6-tert-butyl-8-méthyl-2H-chromén-3-yl) hepta-2,4,6-triénoïque

Point de fusion : 225-227°C
   (cristaux jaunes, solvant : THF/éthanol, 80:20)

### Exemple 13 : Acide (2E,4E,6E)-7-(6,8-ditert-butyl-2H-chromén-3-yl)hepta-2,4,6-triénoïque

Point de fusion : 166-168°C
   (cristaux jaunes, solvant : THF)

### Exemple 14 : Acide (2E,4E,6E)-7-(6-méthyl-8-chloro-2H-chromén-3-yl)hepta-2,4,6-triénoïque

Point de fusion : 248-250°C
   (cristaux oranges, solvant : THF/eau, 80:20)

### Exemple 15 : Acide (2E,4E,6E)-7-(6-chloro-8-méthyl-2H-chromén-3-yl)hepta-2,4,6-triénoïque

Point de fusion : 208-210°C
   (cristaux oranges, solvant : THF/eau, 80:20)

### Exemple 16 : Acide (2E,4E,6E)-7-(2,2-diméthyl-2H-chromén-3-yl)hepta-2,4,6-triénoïque

Point de fusion : 180-182°C
   (cristaux oranges, solvant : THF)

### Exemple 17 : Acide (2E,4E,6E)-7-(2,2-diméthyl-6-méthoxy-2H-chromén-3-yl)hepta-2,4,6-triénoïque

Point de fusion : 154-156°C
   (cristaux oranges, solvant : THF/eau, 80:20)

### Exemple 18 : Acide (2E,4E,6E)-7-(2,2,6,8-tétraméthyl-2H-chromén-3-yl)hepta-2,4,6-triénoïque

Point de fusion : 200-202°C
   (cristaux jaunes, solvant : THF/eau, 80:20)

### Exemple 19 : Acide (2E,4E,6E)-7-(2,2,8-triméthyl-6-chloro-2H-chromén-3-yl)hepta-2,4,6-triénoïque

Point de fusion : 190-192°C
   (cristaux jaunes, solvant : THF)

### Exemple 20 : Acide (2E,4E,6E)-7-(6,8-dichloro-2H-chromén-3-yl)hepta-2,4,6-triénoïque

Point de fusion : 280°C
   (cristaux oranges, solvant : THF/éther de pétrole, 80:20)

### Exemple 21 : Acide (2E,4E,6E)-7-(6-phényl-2H-chromén-3-yl)hepta-2,4,6-triénoïque

### Etape A : 5-Bromo-2-hydroxybenzaldéhyde

Composé préparé de manière identique au composé décrit exemple 1, étape A.

### Etape B : 5-Phényl-2-hydroxybenzaldéhyde

A une solution de 19,7 g (98 mmoles) de 5-bromo-2-hydroxybenzaldéhyde obtenu dans l'étape A et de 64 g (149 mmoles) de tétraphénylétain dans 220 ml de toluène séché sur tamis moléculaire 4Å, sont ajoutés en une seule fois 0,97 g (1,28 mmoles) de trans-benzyl(chloro)bis(triphénylphosphine)palladium(II). Le mélange est porté à reflux pendant 24 heures. Après retour à température ambiante, la solution est filtrée sur Célite. Au filtrat sont ajoutés 20 ml d'une solution méthanolique de fluorure de potassium à 20 %. Après 5 minutes d'agitation et filtration, le filtrat est concentré sous pression réduite. Le solide est repris par 60 ml de dichlorométhane et la solution à nouveau filtrée. La phase organique est lavée par 20 ml d'eau, récupérée puis séchée sur sulfate de magnésium. Après élimination du solvant par distillation et filtration, le solide résiduel est soumis à nouveau et deux fois consécutivement au même traitement.

### Etape C : Acide (2E,4E,6E)-7-(6-phényl-2H-chromén-3-yl)hepta-2,4,6-triénoïque

Le 5-phényl-2-hydroxybenzaldéhyde obtenu dans l'étape B est soumis, de manière analogue, aux traitements décrits dans l'exemple 1, étapes B à G, pour obtenir l'acide attendu. Point de fusion : 260°C (solvant de cristallisation : THF/eau, 90:10)

### Exemple 22 : (1E,3E,5E)-[6-(6-Bromo-2H-chromén-3-yl)hexa-1,3,5-triène]-1-phosphonate de diéthyle

### Etape A : (2E,4E)-5-(6-Bromo-2H-chromén-3-yl)-penta-2,4-diénal

Le 5-bromo-2-hydroxybenzaldéhyde obtenu dans l'exemple 21, étape A est soumis aux traitements décrits dans l'exemple 1, étapes B à E.

### Etape B : (1E,3E,5E)-[6-(6-Bromo-2H-chromén-3-yl)-hexa-1,3,5-triène]-1-phosphonate de diéthyle

La synthèse de cette étape est analogue à celle de l'exemple 1, étape F, en utilisant le diéthylphosphonométhylènephosphonate de diéthyle au lieu du diéthylphosphonoacétate d'éthyle.

### Exemple 23 : (1E,3E,5E)-[6-(6-Adamantanyl-2H-chromén-3-yl)-hexa-1,3,5-triène]-1-phosphonate de diéthyle

Composé obtenu de manière similaire à celui obtenu dans l'exemple 21, étapes A et B, puis exemple 22, étape B.

### Exemple 24 : (2E,4E,6E)-7-(6,8-Di-adamantanyl-2H-chromén-3-yl)-hepta-2,4,6-triénoate d'éthyle

### Etape A : 5,7-Di-adamantanyl-2-hydroxybenzaldéhyde

Intermédiaire préparé de manière analogue à celui décrit exemple 21, étapes A et B.

### Etape B : (2E,4E,6E)-7-(6,8-Di-adamantanyl-2H-chromén-3-yl)-hepta-2,4,6-triénoate d'éthyle

Le 5,7-di-adamantanyl-2-hydroxybenzaldéhyde obtenu à l'étape A est soumis de manière analogue aux traitements décrits dans l'exemple 1, étapes B à F pour obtenir le produit attendu.

### Exemple 25 : (2E,4E,6E)-3-Méthyl-7-(6-phényl-2H-chromén-3-yl)octa-2,4,6-triénoate d'éthyle

### Etape A : 3-Acétyl-6-bromo-2H-chromène

Une solution hétérogène de 100 g (497 mmoles) de 5-bromo-2-hydroxybenzaldéhyde et de 13,8 g (10 mmoles) de carbonate de potassium (préalablement finement broyé et séché à 90°C) dans 220 ml de butan-2-one est portée à reflux. Une solution de 35,31 g (soit 42 ml, 497 mmoles) de but-3-én-2-one dans 40 ml de butan-2-one est alors additionnée goutte à goutte, pendant 15 à 20 minutes. Après 4 heures de reflux, 13,8 g (10 mmoles) de carbonate de potassium sont ajoutés. Le mélange est laissé 16 heures à reflux. Le milieu réactionnel étant revenu à température ambiante, le solide est séparé par filtration puis lavé par 2 fois 50 ml de butan-2-one. Le filtrat est ensuite concentré et le résidu purifié par chromatographie sur colonne de silice. (Eluant : dichlorométhane/cyclohexane 1/1). Après recristallisation dans l'éther diisopropylique, 73 g d'un solide jaunâtre sont obtenus.
Rendement : 58 %
Point de fusion : 70°C

### Etape B : 3-Acétyl-6-phényl-2H-chromène

A une solution de 25 g (98 mmoles) de 3-acétyl-6-bromo-2H-chromène obtenu dans l'étape A et de 64 g (149 mmoles) de tétraphénylétain dans 220 ml de toluène séché sur tamis moléculaire 4Å, sont ajoutés en une seule fois 0,97 g (1,28 mmoles) de trans-benzyl(chloro)bis(triphénylphosphine)palladium(II). Le mélange est porté à reflux pendant 24 heures. Après retour à température ambiante, la solution est filtrée sur Célite. Au filtrat sont ajoutés 20 ml d'une solution méthanolique de fluorure de potassium à 20 %. Après 5 minutes d'agitation et filtration, le filtrat est concentré sous pression réduite. Le solide est repris par 60 ml de dichlorométhane et la solution à nouveau filtrée. La phase organique est lavée par 20 ml d'eau, récupérée puis séchée sur sulfate de magnésium. Après élimination du solvant par distillation et filtration, le solide résiduel est soumis à nouveau et deux fois consécutivement au même traitement. 12 g d'un solide jaune sont obtenus.
Rendement : 42 %
Point de fusion : 126°C

### Etape C : 3-Hydroxy-3-(6-phényl-2H-chromén-3-yl)butanoate d'éthyle

4,35 g (0,0665 At-g) de zinc activé sont recouverts par 12 ml de diméthoxyméthane, puis 1 ml (9 mmoles) de bromoacétate d'éthyle pur est ajouté sous atmosphère d'argon. Après induction de la réaction par chauffage, une solution de 6,38 ml (57,5 mmoles) de bromoacétate d'éthyle dans 20 ml de diméthoxyméthane est additionnée de façon à entretenir le reflux. Après complète consommation du zinc et retour du milieu réactionnel à température ambiante, une solution de 11,1 g (43,5 mmoles) de 3-acétyl-6-phényl-2*H*-chromène dans 60 ml de tétrahydrofurane anhydre est additionnée goutte à goutte pendant 10 à 15 minutes. Après une heure d'agitation à température ambiante, le mélange refroidi à +4°C est traité par 25 ml d'une solution d'acide sulfurique à 10 %. Après addition de 70 ml d'éther diéthylique, la phase aqueuse est séparée, la phase éthérée est lavée par 3 x 20 ml d'eau, puis séchée sur sulfate de magnésium. Les solvants sont évaporés sous pression réduite pour obtenir un solide utilisé sans autre purification dans l'étape suivante.

### Etape D : 3-(6-Phényl-2H-chromén-3-yl)but-2-énoate d'éthyle

La totalité du solide obtenu dans l'étape C est solubilisée dans 70 ml de toluène, puis traitée par 1,25 ml (13,3 mmoles) d'oxychlorure de phosphore. Le mélange est porté à reflux pendant 1 heure. Après retour à température ambiante, le milieu réacitonnel est versé sur 60 ml d'eau glacée, puis mélangé à 100 ml d'éther diéthylique. Le traitement habituel de la phase organique fournit, après purification sur colonne de silice (éluant dichlorométhane) 12,36 g d'un solide jaune recristallisé dans l'éther diisopropylique.
Rendement : 87 %
Point de fusion : 97°C

### Etape E : 3-(6-Phényl-2H-chromén-3-yl)but-2-én-1-ol

A une solution de 4,95 g (37 mmoles) de chlorure d'aluminium anhydre sublimé et de 50 ml d'éther diéthylique, maintenue à 0°C et sous argon, 3,47 g (98 mmoles) d'hydrure double de lithium et d'aluminium sont ajoutés. Le complexe formé est agité à 0°C pendant 20 minutes, puis une solution de 11,9 g (37,1 mmoles) d'ester obtenu dans l'étape D dans 130 ml d'un mélange d'éther diéthylique et de tétrahydrofurane (10/3) est ajoutée rapidement. L'ensemble est maintenu 45 minutes à 0°C. L'excès d'hydrure est décomposé par addition successive de 10 ml d'acide chlorhydrique 0,4 N, 10 ml d'acide chlorhydrique 1N et 20 ml d'acide chlorhydrique 4N. Après dilution par 100 ml d'eau, le milieu réactionnel est extrait par de l'éther diéthylique (500 à 2000 ml). Les phases éthérées sont traitées de manière habituelle et livrent un liquide jaune pâle utilisé directement dans l'étape suivante sans purification supplémentaire.

### Etape F : 3-(6-Phényl-2H-chromén-3-yl)but-2-énal

A une solution de 9,4 g (33,7 mmoles) de l'alcool obtenu dans l'étape E dans 160 ml de dichlorométhane anhydre sont ajoutés en trois fois à 15 minutes d'intervalle 29,36 g (33,7 mmoles) de dioxyde de manganèse. Après une heure sous agitation, le mélange réactionnel est filtré sur Célite et le dichlorométhane éliminé par évaporation sous pression réduite. Le produit brut est recristallisé dans l'éther diisopropylique pour donner finalement 8,27 g d'un solide jaune.
Rendement : 89 %
Point de fusion : 147°C

### Etape G : (2E,4E,6E)-3-Méthyl-7-(6-phényl-2H-chromén-3-yl)octa-2,4,6-triénoate d'éthyle

A une solution de 9,49 g (36 mmoles) de (*E*)-4-diéthoxyphosphoryl-3-méthylbut-2-énoate d'éthyle dans 70 ml de tétrahydrofurane anhydre est ajouté, sous argon, par petites portions 0,905 g (37,7 mmoles) d'hydrure de sodium. Après 30 minutes d'agitation, une solution de 8,27 g (30 mmoles) de l'aldéhyde obtenu dans l'étape F dans 140 ml de tétrahydrofurane est ajoutée goutte à goutte pendans 30 minutes.

Après une heure d'agitation, le milieu réactionnel est refroidi à 10°C et additionné goutte à goutte de 100 ml d'eau distillée, puis de 45 ml d'une solution d'acide chlorhydrique 0,1 N et enfin de 140 ml d'eau. Après une heure au repos à +10°C, le solide jaune est séparé par filtration et lavé abondamment à l'eau. Après séchage à 70°C sous vide phosphorique, 11,5 g d'ester triénique sous forme d'un mélange d'isomères (2*E*,4*E*,6*E*) et (2*Z*,4*E*,6*E*) sont récupérés, puis recristallisés deux fois dans l'éther diéthylique et l'éthanol pour livrer finalement 7,34 g d'un solide jaune.
Rendement : 63 %
Point de fusion : 159°C

### Exemple 26 : Acide (2E,4E,6E)-3-méthyl-7-(6-phényl-2H-chromén-3-yl)octa-2,4,6-triénoïque

A une solution de 7,34 g (19 mmoles) de l'ester obtenu dans l'exemple 25, dans un mélange de 310 ml d'éthanol et de 140 ml d'eau distillée, sont ajoutés 44 ml d'hydroxyde de sodium à 35 %. Le milieu réactionnel est ensuite porté à reflux pendant 2 heures. La solution refroidie à +10°C est acidifiée à l'aide d'une solution d'acide chlorhydrique 4N jusqu'à pH = 4. Après addition de 80 ml d'eau, le mélange est agité une heure à +10°C. Après filtration, le solide est rincé à l'eau et séché à l'étuve, à 70°C sous vide phosphorique. Après recristallisation dans l'éthanol, 4,6 g d'un solide jaune sont obtenus.
Rendement : 67 %
Point de fusion : 249°C (décomposition)

### Exemple 27 : (2Z,4E,6E)-3-Méthyl-7-(6-phényl-2H-chromén-3-yl)octa-2,4,6-triénoate d'éthyle

Les eaux mères de cristallisation du composé décrit dans l'exemple 25, étape G, sont évaporées sous pression réduite, et le solide ainsi obtenu est chromatographié sur colonne de silice (éluant : dichlorométhane). 2,8 g d'un solide jaune sont obtenus.
Rendement : 24,3 %
Point de fusion : 159°C (recristallisé dans l'éthanol)

### Exemple 28 : Acide (2Z,4E,6E)-7-(6-phényl-2H-chromén-3-yl)-3-méthylocta-2,4,6-triénoïque

Le protocole décrit dans l'exemple 26 est appliqué au composé obtenu dans l'exemple 27.
Rendement : 78 %
Point de fusion : > 250°C (décomposition)

Les esters suivants ont été obtenus de manière analogue au procédé décrit dans l'exemple 1.

### Exemple 29 : (2E,4E,6E)-7-(6-Adamantanyl-2H-chromén-3-yl)-3-méthylocta-2,4,6-triénoate d'éthyle

### Exemple 30 : (2E,4E,6E)-7-[6-(2-Pyridyl)-2H-chromén-3-yl]-3-méthylocta-2,4,6-triénoate d'éthyle

### Exemple 31 : (2E,4E,6E)-7-[6-(2-Thiényl)-2H-chromén-3-yl]-3-méthylocta-2,4,6-triénoate d'éthyle

Les esters suivants ont été obtenus de manière analogue au procédé décrit dans l'exemple 25, hormis l'étape B, à partir des hydroxybenzaldéhydes appropriés.

### Exemple 32 : (2E,4E,6E)-7-(6-Méthyl-2H-chromén-3-yl)-3-méthylocta-2,4,6-triénoate d'éthyle

Point de fusion : 135°C
   (solvant de cristallisation : éthanol)

### Exemple 33 : (2E,4E,6E)-7-(6-Tert-butyl-2H-chromén-3-yl)-3-méthylocta-2,4,6-triénoate d'éthyle

Point de fusion : 129°C
   (solvant de cristallisation : éthanol)

### Exemple 34 : (2E,4E,6E)-7-(6-Trifluorométhyl-2H-chromén-3-yl)-3-méthylocta-2,4,6-triénoate d'éthyle

Point de fusion : 163-165°C
   (solvant de cristallisation : éthanol)

### Exemple 35 : (2E,4E,6E)-7-(6,8-Diméthyl-2H-chromén-3-yl)-3-méthylocta-2,4,6-triénoate d'éthyle

Point de fusion : 122°C
   (solvant de cristallisation : éther isopropylique)

### Exemple 36 : (2E,4E,6E)-7-(6-Tert-butyl-8-méthyl-2H-chromén-3-yl)-3-méthylocta-2,4,6-triénoate d'éthyle

Point de fusion : 73°C
   (solvant de cristallisation : heptane)

### Exemple 37 : (2E,4E,6E)-7-(6,8-Ditert-butyl-2H-chromén-3-yl)-3-méthylocta-2,4,6-triénoate d'éthyle

Point de fusion : 130-131°C
   (solvant de cristallisation : éther diisopropylique)

### Exemple 38 : (2E,4E,6E)-7-(7-Méthoxy-2H-chromén-3-yl)-3-méthylocta-2,4,6-triénoate d'éthyle

### Exemple 39 : (2E,4E,6E) 7-(2,2-Diméthyl-6-éthyl-2H-chromén-3-yl)-3-méthylocta-2,4,6-triénoate d'éthyle

Les acides suivants ont été préparés de manière analogue au composé décrit dans l'exemple 26, à partir de leur ester respectif :

### Exemple 40: Acide (2E,4E,6E)-7-(6-méthyl-2H-chromén-3-yl)-3-méthylocta-2,4,6-triénoïque

Point de fusion : 244°C (décomposition)
   (solvant de cristallisation : éthanol)

### Exemple 41 : Acide (2E,4E,6E)-7-(6-tert-butyl-2H-chromén-3-yl)-3-méthylocta-2,4,6-triénoïque

Point de fusion : 229-231°C (décomposition)
   (solvant de cristallisation : acétate d'éthyle)

### Exemple 42 : Acide (2E,4E,6E)-7-(6-trifluorométhyl-2H-chromén-3-yl)-3-méthylocta-2,4,6-triénoïque

Point de fusion : 245-246°C
   (solvant de cristallisation : éthanol/acétate d'éthyle)

### Exemple 43 : Acide (2E,4E,6E)-7-(6,8-diméthyl-2H-chromén-3-yl)-3-méthylocta-2,4,6-triénoïque

Point de fusion : 233-235°C (décomposition)
   (solvant de cristallisation : acétate d'éthyle)

### Exemple 44 : Acide (2E,4E,6E)-7-(6-tert-butyl-8-méthyl-2H-chromén-3-yl)-3-méthylocta-2,4,6-triénoïque

Point de fusion : 227-228°C
   (solvant de cristallisation : acétate d'éthyle)

### Exemple 45 : Acide (2E,4E,6E)-7-(6,8-ditert-butyl-2H-chromén-3-yl)-3-méthylocta-2,4,6-triénoïque

Point de fusion : 240-241°C
   (solvant de cristallisation : éthanol)

Les deux exemples suivants sont réalisés selon le même mode opératoire que l'exemple 25, le réactif de Horner étant cette fois le (3-diéthoxyphosphoryl-2-méthylprop-1-ène)-1-phosphonate de diéthyle :

### Exemple 46 : (1E,3E,5E)-[2-Méthyl-6-(6,8-diméthyl-2H-chromén-3-yl)hepta-1,3,5-triène]-1-phosphonate de diéthyle

Point de fusion : 95-96°C
   (solvant de cristallisation : éther de pétrole)

### Exemple 47 : (1E,3E,5E)-[2-Méthyl-6-(6-méthyl-8-tert-butyl-2H-chromén-3-yl)hepta-1,3,5-triéne]-1-phosphonate de diéthyle

### Exemple 48 : Acide (1E,3E,5E)-[2-méthyl-6-(2,2-diméthyl-7-méthoxy-2H-chromén-3-yl)hepta-1,3,5-triène]-1-phosphonique

Composé préparé selon le protocole décrit dans l'exemple 26, à partir du phosphonate de diéthyle correspondant.

### Exemple 49 : Acide (2Z,4E,6E)-3-méthyl-7-(6-adamantan-1-yl-2H-chromén-3-yl)octa-2,4,6-triénoïque

Composé obtenu à partir de l'ester décrit à l'exemple 29, selon le procédé de l'exemple 26.
Point de fusion : 247°C (Décomposition)

### Exemple 50 : (2E,4E,6E)-3-Méthyl-7-(6-adamantan-1-yl-2H-chromén-3-yl)octa-2,4,6-triénoate d'éthyle

En procédant comme décrit à l'exemple 27, à partir des eaux mères obtenues à l'exemple 29, le produit titre est isolé.
Point de fusion : 178-179°C

### Exemple 51 : Acide (2E,4E,6E)-3-méthyl-7-(6-adamantan-1-yl-2H-chromén-3-yl)octa-2,4,6-triénoïque

Le composé obtenu dans l'exemple 50 est saponifié selon la technique décrite pour l'exemple 26.
Point de fusion : >250°C

### ETUDE PHARMACOLOGIQUE

### Exemple A : Anti-résorption osseuse - 1

Des propriétés anti-résorbantes osseuses ont été mises en évidence sur des calvaria de souris selon un modèle inspiré de la méthode décrite par Reynolds et Dingle ("A sensitive in vitro method for studying the induction and inhibition of bone resorption", Calc. Tiss. Res., 4, (1970), 339-349). Les composés ont été étudiés sur un tissu en hyperrésorption provoquée par addition de 5.10⁻⁸ M d'acide rétinoïque. Les résultats obtenus sont présentés dans le tableau I suivant :

**- Tableau I -**

| Anti-résorption osseuse | | |
|---|---|---|
| Exemple | Concentration (µM) | Inhibition de la résorption (%) |
| 25 | 50 | 15 |
| 26 | 10 | 11 |
| | 50 | 29 |
| 27 | 10 | 11 |
| 32 | 10 | 14 |
| | 50 | 12 |
| 33 | 10 | 3 |
| 35 | 10 | 15 |
| | 50 | 32 |
| Référence : Etrétinate | 50 | 11 |

Cette activité s'exerce de façon comparable lorsque l'hyperrésorption est provoquée par addition de parathormone (PTH) (500 ng/ml).

| Exemple | Hyperrésorption à l'acide rétinoïque | Hyperrésorption à la PTH |
|---|---|---|
| 35 (7,5 µM) | 9 % | 12 % |

### Exemple B : Anti-résorption osseuse - 2

Etude in vivo, selon la méthode de mesure décrite par Konig, Muhlbauer et Fleisch ("Tumor necrosis factor and interleukine-1 stimulate bone resorption in vivo as mesured by urinary [³H] tetracycline excretion from prelabled mice", J. Bone Min. Res., 3, (1988), 621-627). Une étude a été réalisée chez des souris pour lesquelles un état d'hyperrésorption a été provoqué par une alimentation pauvre en calcium (0,4 %). Un frein à l'hyperrésorption a été observé après traitement par un des composés de l'invention contrairement à l'étrétinate qui tend à augmenter l'excrétion urinaire de tétracycline tritiée. Les résultats sont présentés dans le tableau II suivant :

**- Tableau II -**

| - % de variation de l'excrétion urinaire de [³H]-tétracycline - | | | |
|---|---|---|---|
| | Dose orale (mg/kg) | | |
| | 50 | 100 | 200 |
| Exemple 26 | - 10 % | - 10 % | - 12 % |
| Etrétinate | + 11 % | + 3 % | - |

### Exemple C : Formation du tissu ostéoïde

Un effet facilitant la formation de tissu ostéoïde (incorporation de proline tritiée du milieu dans le tissu osseux de calvaria de souris) a également été observé avec certains composés :

| Exemple | Concentration (µM) | Stimulation de la formation |
|---|---|---|
| 26 | 10 | + 7 % |
| 35 | 10 | + 11 % |

### Exemple D : Activité anti-inflammatoire

Les composés de la présente invention excercent une activité inhibitrice sur la libération d'acide oléique tritié préalablement incorporé à des membranes *d'Escherichia coli* sous l'effet de la phospholipase A₂ (PLA₂) pancréatique de porc selon la technique décrite par Frason, Patriarca et Elsbach (J. Lipid. Res., 15, (1974), 380-388). Les résultats sont exposés dans le tableau III ci-dessous :

**- Tableau III -**

| Test sur les membranes d'*Escherichia coli* | | | |
|---|---|---|---|
| Exemple | IC₅₀ (µM) | Exemple | IC₅₀ (µM) |
| 5 | 7,5 | 20 | 8 |
| 6 | 9 | 25 | 5 |
| 11 | 6 | 26 | 5 |
| 13 | 3 | 27 | 7,5 |
| 14 | 20 | 32 | 7,5 |
| 15 | 10 | 35 | 5 |
| 18 | 30 | 36 | 5 |
| 19 | 30 | 41 | 4 |

### Exemple E : Test sur les polymorphonucléaires de rat

Sur un modèle cellulaire impliquant la PLA₂ endogène membranaire, les composés de l'invention ont également exercé un effet inhibiteur sur la libération de l'acide arachidonique par leur action sur les polymorphonucléaires (PLN) isolés de rat dont les phospholipides membranaires ont été marqués à l'acide arachidonique tritié. Le degré d'activité des produits de l'invention est apprécié par mesure de la radioactivité libérée après stimulation par l'ionophore A 12187 selon la technique décrite par Sakson, Raz, Denny, Wyche et Needleman (Prostaglandins, 14, (1977), 853, sqq.).

Les résultats sont indiqués dans le tableau IV ci-dessous :

**- Tableau IV -**

| Test sur les PMNs de rat | |
|---|---|
| Exemple | IC₅₀ (µM) |
| 5 | 30 |
| 6 | 10 |
| 11 | 5 |
| 13 | 5 |
| 14 | 50 |
| 15 | 50 |
| 18 | 30 |
| 19 | 50 |
| 20 | 30 |

## Revendications

1. Composés de formule générale (I) : 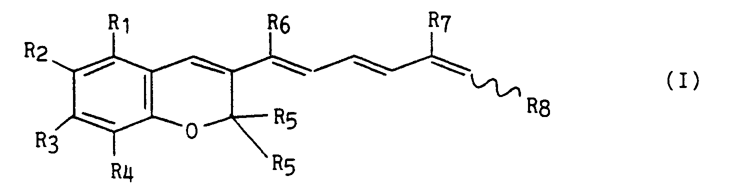 dans laquelle :
- R₁, R₂, R₃, R₄, identiques ou différents, représentent :
- un atome d'hydrogène,
- un atome d'halogène,
- un radical phényle substitué ou non,
- un radical adamantyle,
- un radical hétérocyclique substitué ou non, contenant 5 ou 6 atomes, choisi parmi les radicaux furyle, pyrrolyle, pyrrolidinyle, thiényle, pyridyle, pipéridyle, indolyle et quinolyle,
- un radical R' ou
- un radical OR',
- R' étant un groupement alkyle linéaire ou ramifié contenant de 1 à 6 atomes de carbone, éventuellement substitués par un ou plusieurs groupements hydroxy, et/ou un ou plusieurs atomes d'halogène,
- R₅, R₆ et R₇, identiques ou différents, sont choisis parmi un atome d'hydrogène et un groupement alkyle linéaire ou ramifié contenant de 1 à 6 atomes de carbone,
- R₈ est choisi parmi les groupements C(O)OR₉, P(O)(OR₉)₂ et CONHR₁₀ dans lesquels R₉ est un atome d'hydrogène, un groupement alkyle linéaire ou ramifié contenant de 1 à 6 atomes de carbone ou un résidu d'un amino-sucre, et R₁₀ représente un atome d'hydrogène, un groupement alkyle linéaire ou ramifié contenant de 1 à 6 atomes de carbone ou un radical aryle éventuellement substitué,
la liaison indéfinie portant R₈ étant une liaison simple conférant une configuration E ou Z à la double liaison qui la supporte,
avec la restriction que lorsque R₅ représente l'hydrogène et R₆ et R₇ représentent chacun le groupement méthyle, et R₈ représente le groupement C(O)OR9 tel que précédemment défini, alors au moins un des substituants R₁, R₂, R₃ ou R₄ est différent de l'hydrogène d'un halogène d'un radical R', et d'un radical OR', R' étant tel que défini précédemment,
leurs stéréoisomères ainsi que leurs éventuels sels d'addition à un acide ou à une base, pharmaceutiquement acceptable.

2. Composé selon la revendication 1 qui est l'acide (2*E*,4*E*,6*E*)-7-(2,2-diméthyl-6-méthoxy-2*H*-chromén-3-yl)hepta-2,4,6-triénoïque,
ainsi que ses sels d'addition à une base, pharmaceutiquement acceptables.

3. Composé selon la revendication 1 qui est l'acide (2*E*,4*E*,6*E*)-7-(6-tert-butyl-2*H*-chromén-3-yl)hepta-2,4,6-triénoïque,
ainsi que ses sels d'addition à une base, pharmaceutiquement acceptables.

4. Composé selon la revendication 1 qui est l'acide (2*E*,4*E*,6*E*)-7-(6,8-ditert-butyl-2*H*-chromén-3-yl)hepta-2,4,6-triénoïque,
ainsi que ses sels d'addition à une base, pharmaceutiquement acceptables.

5. Composé selon la revendication 1 qui est l'acide (2*E*,4*E*,6*E*)-3-méthyl-7-(6-phényl-2*H*-chromén-3-yl)octa-2,4,6-triénoïque,
ainsi que ses sels d'addition à une base, pharmaceutiquement acceptables.

6. Composé selon la revendication 1 qui est le (2*Z*,4*E*,6*E*)-3-méthyl-7-(6-phényl-2*H*-chromén-3-yl)octa-2,4,6-triénoate d'éthyle.

7. Composé selon la revendication 1 qui est le (1*E*,3*E*,5*E*)-[2-méthyl-6-(6,8-diméthyl-2*H*-chromén-3-yl)hepta-1,3,5-triène]-1-phosphonate de diéthyle.

8. Procédé de préparation des composés de formule (I) caractérisé en ce que les composés de formule (II) : 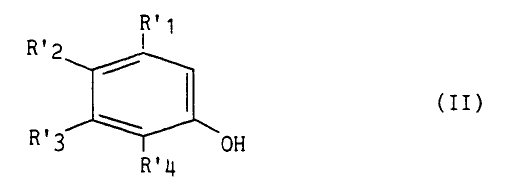 dans laquelle R'₁, R'₂, R'₃ et R'₄, identiques ou différents, représentent un atome d'hydrogène ou d'halogène, un groupement alkyle ou alkoxy linéaire ou ramifié contenant de 1 à 6 atomes de carbone, éventuellement substitués par un ou plusieurs groupements OH, et/ou par un ou plusieurs atomes d'halogène,
est transformé en composé de formule (III) : 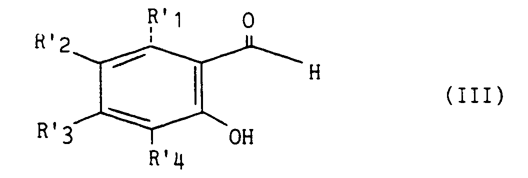 dans laquelle R'₁, R'₂, R'₃ et R'₄ ont les mêmes significations que précédemment,
selon la réaction de Sommelet, par action du para-formaldéhyde en présence d'hexaméthylènetétramine, à une température de 100°C, puis acidification,
ou bien par action d'un chlorure métallique, en présence d'une alkylamine, avec le trioxyméthylène, également à 100°C, composé de formule (III) qui est ensuite cyclisé par action d'un composé de formule (IV) : 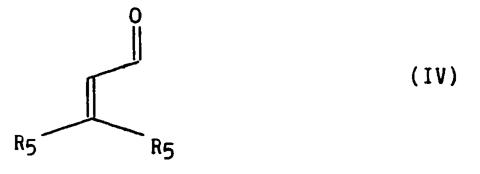 dans laquelle R₅ est tel que défini dans la formule (I), en présence d'un carbonate alcalin, dans un solvant polaire à reflux, pour conduire au composé de formule (V) : 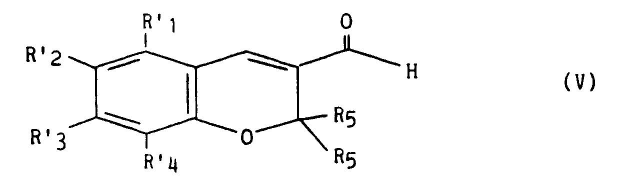 dans laquelle R'₁, R'₂, R'₃, R'₄ et R₅ sont tels que définis précédemment,
composé de formule (V) dont les groupements R'₁, R'₂, R'₃ et R'₄ lorsque l'un ou plusieurs d'entre eux représentent un atome d'halogène peuvent être substitués par un radical phényle, phényle substitué, adamantyle, ou un radical hétérocyclique, par réaction avec un tétra-phényl-, -aryl-, -hétérocyclyl-, ou -hétéroaryl-étain correspondant en présence d'un catalyseur à base de palladium, dans le toluène à reflux, de façon à obtenir l'ensemble des composés de formule (VI) : 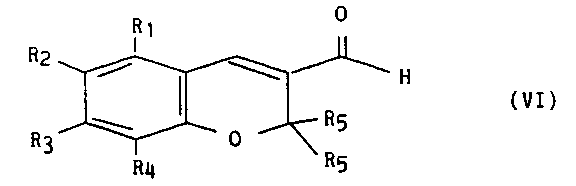 dans laquelle R₁, R₂, R₃, R₄ et R₅ ont les mêmes significations que précédemment,
qui, par réaction de Wittig à l'aide d'un composé de formule (VII) : 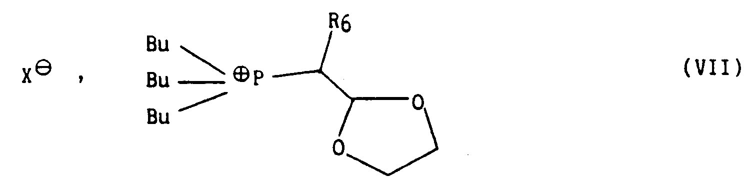 où X est un atome d'halogène et R₆ est tel que défini précédemment,
puis hydrolyse acide, conduisent au composé de formule (VIII) : 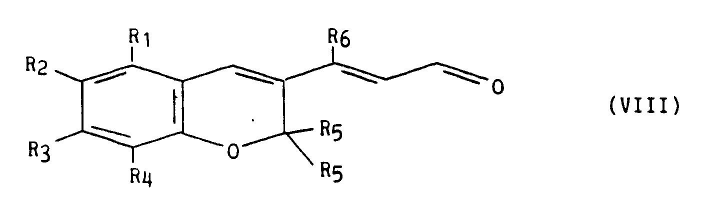 où R₁, R₂, R₃, R₄, R₅ et R₆ sont tels que définis précédemment,
composé de formule (VIII) qui, après une seconde réaction de Wittig, dans les mêmes conditions, avec un composé de formule (VII') : 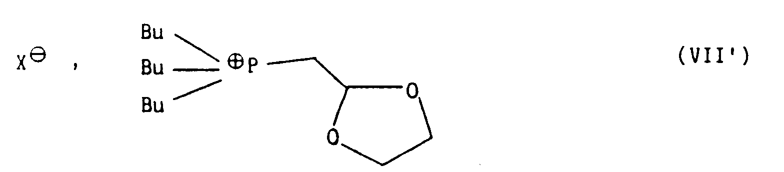 dans laquelle X est tel que défini précédemment,
est transformé en composé de formule (IX) : 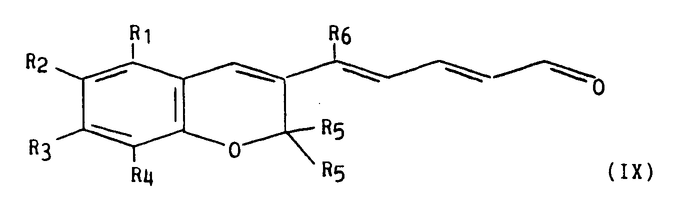 dans laquelle les groupements R₁ à R₆ sont tels que définis précédemment,
qui est finalement traité par un composé de formule (Xa) ou (Xb) : 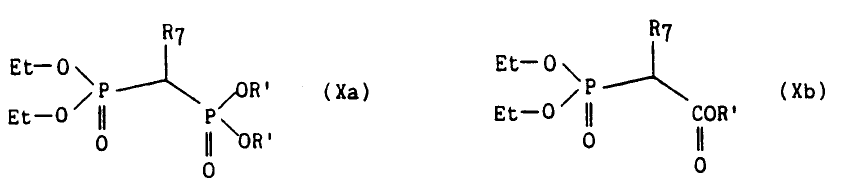 dans laquelle R₇ et R' sont tels que définis précédemment,
pour conduire respectivement aux composés de formules (Ia) et (Ib) : 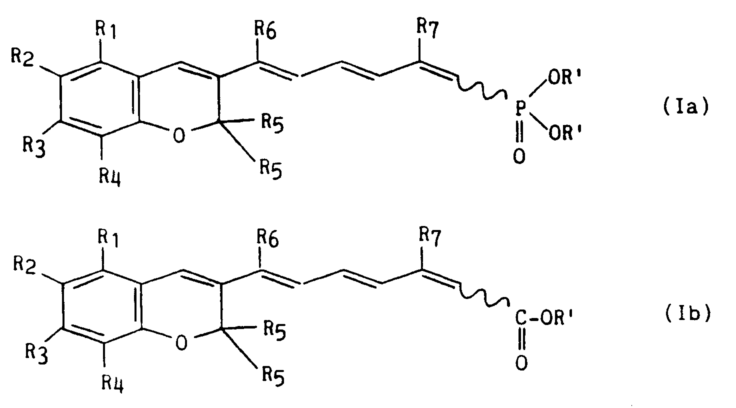 dans lesquelles les groupements R₁ à R₇ et R' sont tels que définis précédemment,
composé de formule (Ia) qui, traité en milieu basique ou acide, permet d'obtenir le composé de formule (Ic) : 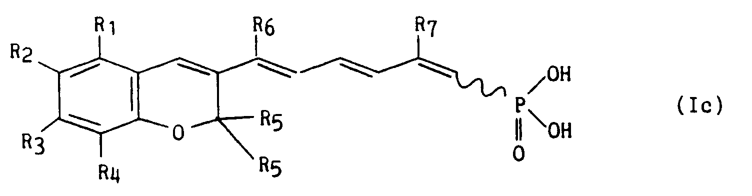 dans laquelle les groupements R₁ à R₇ sont tels que définis précédemment,
composé de formules (Ib) qui peut être saponifié en composé de formule (Id) : 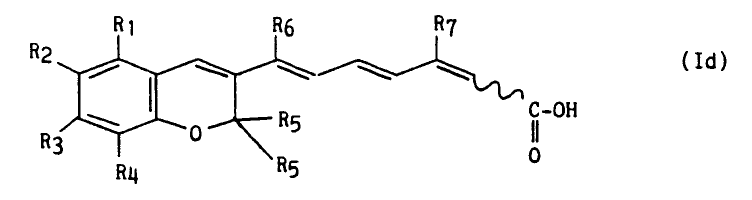 dans laquelle les groupements R₁ à R₇ sont tels que définis précédemment,
qui, à son tour, après transformation en son chlorure d'acyle correspondant, et traitement par une amine de formule (XI) :
R₁₀-NH₂ (XI)
dans laquelle R₁₀ est tel que défini précédemment,
conduit au composé de formule (Ie) : 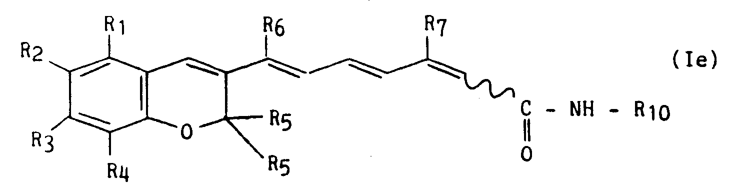 dans laquelle les groupements R₁ à R₇ et R₁₀ sont tels que définis précédemment,
l'ensemble des composés de formule (Ia) à (Ie) formant l'ensemble des composés de formule générale (I) que l'on purifie le cas échéant selon une technique classique de purification, dont on sépare, si on le souhaite, les stéréoisomères et les isomères optiques par une technique classique de séparation et que l'on transforme si on le souhaite, en leurs sels d'addition à un acide ou à une base pharmaceutiquement acceptable.

9. Compositions pharmaceutiques contenant comme principe actif au moins un composé selon l'une des revendications 1 à 7 en combinaison avec un ou plusieurs excipients ou véhicules inertes, non toxiques et pharmaceutiquement acceptables.

10. Compositions pharmaceutiques selon la revendication 9 contenant au moins un principe actif selon l'une des revendications 1 à 7 possédant des propriétés anti-résorbantes osseuses, génératrices de tissu osseux et utiles dans le traitement de l'ostéoporose et des affections inflammatoires et en particulier dans les inflammations chroniques ostéopéniantes généralisées, ou localisées.

11. Utilisation des composés de formule (I') : 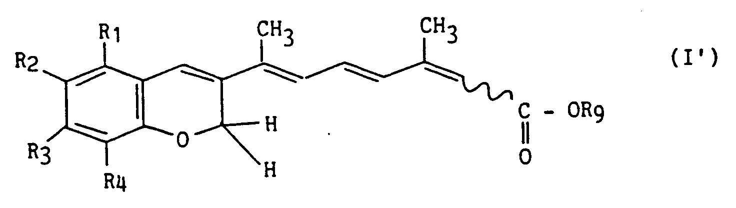 dans laquelle :
- R₁, R₂, R₃ et R₄ identiques ou différents sont choisis parmi :
- un atomme d'hydrogène,
- un atome d'halogène,
- un radical R' et
- un radical OR',
- R' étant un groupement alkyle, linéaire ou ramifié, contenant de 1 à 6 atomes de carbone et éventuellement substitués par un ou plusieurs groupements hydroxy, et/ou un ou plusieurs atome d'halogène,
- R₉ est choisi parmi un atome d'hydrogène, un groupement alkyle linéaire ou ramifié contenant de 1 à 6 atomes de carbone ou un résidu d'un amino-sucre,
la liaison indéfinie portant le radical 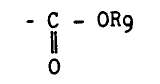 étant une liaison simple conférant une configuration Z ou E à la double liaison qui la supporte,
ainsi qu'à leurs éventuels stéréoisomères et sels d'addition à un acide ou à une base, pharmaceutiquement acceptables,pour l'obtention de compositions pharmaceutiques utilisables
dans le traitement de l'ostéoporose et des affections inflammatoires et en particulier dans les inflammations chroniques ostéopéniantes généralisées ou localisées.

## Claims

1. Compounds of the general formula (I): 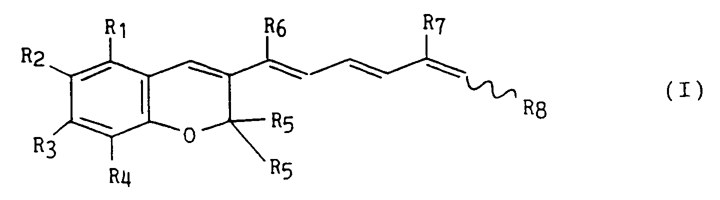 in which:
- R₁, R₂, R₃ and R₄, which are the same or different, represent:
- a hydrogen atom,
- a halogen atom,
- an unsubstituted or substituted phenyl radical,
- an adamantyl radical,
- an unsubstituted or substituted heterocyclic radical containing 5 or 6 atoms, selected from the radicals furyl, pyrrolyl, pyrrolidinyl, thienyl, pyridyl, piperidyl, indolyl and quinolyl,
- a radical R', or
- a radical OR',
- R' being a linear or branched alkyl group containing from 1 to 6 carbon atoms that is optionally substituted by one or more hydroxy groups and/or by one or more halogen atoms,
- R₅, R₆ and R₇, which are the same or different, are selected from a hydrogen atom and a linear or branched alkyl group containing from 1 to 6 carbon atoms,
- R₈ is selected from the groups C(O)OR₉, P(O)(OR₉)₂ and CONHR₁₀ in which R₉ is a hydrogen atom, a linear or branched alkyl group containing from 1 to 6 carbon atoms, or a residue of an amino-sugar, and R₁₀ represents a hydrogen atom, a linear or branched alkyl group containing from 1 to 6 carbon atoms, or an optionally substituted aryl radical,
the non-defined bond carrying R₈ being a single bond conferring an E- or Z-configuration on the double bond supporting it,
with the proviso that when R₅ represents hydrogen and R₆ and R₇ each represents a methyl group and R₈ represents the group C(O)OR₉ as defined above, then at least one of the substituents R₁, R₂, R₃ and R₄ is other than hydrogen, a halogen, a radical R' and a radical OR', R' being as defined above,
their stereoisomers and, where appropriate, their addition salts with a pharmaceutically acceptable acid or base.

2. Compound according to claim 1 which is (2E,4E,6E)-7-(2,2-dimethyl-6-methoxy-2H-chromen-3-yl)hepta-2,4,6-trienoic acid, and its addition salts with a pharmaceutically acceptable base.

3. Compound according to claim 1 which is (2E,4E,6E)-7-(6-tert.-butyl-2H-chromen-3-yl)hepta-2,4,6-trienoic acid, and its addition salts with a pharmaceutically acceptable base.

4. Compound according to claim 1 which is (2E,4E,6E)-7-(6,8-ditert.-butyl-2H-chromen-3-yl)hepta-2,4,6-trienoic acid,
and its addition salts with a pharmaceutically acceptable base.

5. Compound according to claim 1 which is (2E,4E,6E)-3-methyl-7-(6-phenyl-2H-chromen-3-yl)octa-2,4,6-trienoic acid,
and its addition salts with a pharmaceutically acceptable base.

6. Compound according to claim 1 which is ethyl (2Z,4E,6E)-3-methyl-7-(6-phenyl-2H-chromen-3-yl)octa-2,4,6-trienoate.

7. Compound according to claim 1 which is diethyl (1E,3E,5E)-[2-methyl-6-(6,8-dimethyl-2H-chromen-3-yl)hepta-1,3,5-triene]-1-phosphonate.

8. Process for the preparation of compounds of formula (I), characterised in that the compound of formula (II): 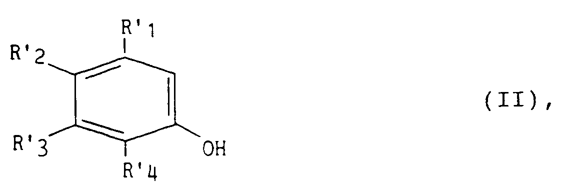 in which R'₁, R'₂, R'₃ and R'₄, which are the same or different, represent a hydrogen atom, a halogen atom, or a linear or branched alkyl or alkoxy group containing from 1 to 6 carbon atoms that is optionally substituted by one or more OH groups and/or by one or more halogen atoms,
is converted into the compound of formula (III): 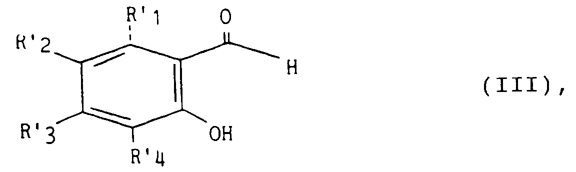 in which R'₁, R'₂, R'₃ and R'₄ have the same meanings as above,
by the Sommelet reaction, by the action of paraformaldehyde in the presence of hexamethylenetetramine, at a temperature of 100°C, followed by acidification,
or alternatively by the action of a metal chloride, in the presence of an alkylamine, with trioxymethylene, likewise at 100°C,
which compound of formula (III) is then cyclised by the action of a compound of formula (IV): 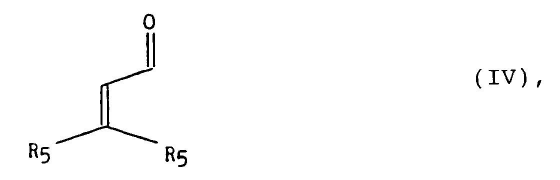 in which R₅ is as defined in formula (I),
in the presence of an alkali metal carbonate, in a polar solvent at reflux, to give the compound of formula (V): 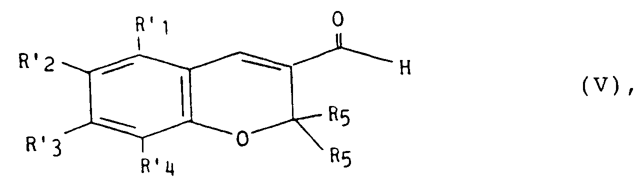 in which R'₁, R'₂, R'₃, R'₄ and R₅ are as defined above,
it being possible for the groups R'₁, R'₂, R'₃ and R'₄ of that compound of formula (V), when one or more of those groups represents a halogen atom, to be replaced by a phenyl radical, a substituted phenyl radical, an adamantyl radical, or by a heterocyclic radical, by reaction with a corresponding tetra-phenyl-, -aryl-, -heterocyclyl- or -heteroaryl-tin, in the presence of a palladium-based catalyst, in toluene at reflux, thereby obtaining all the various compounds of formula (VI): 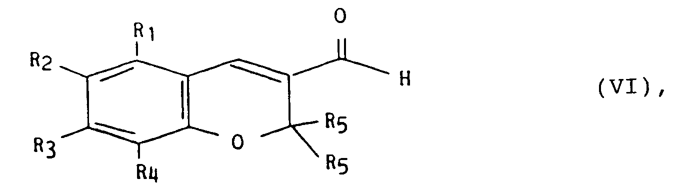 in which R₁, R₂, R₃, R₄ and R₅ have the same meanings as above,
which, by means of a Wittig reaction with the aid of a compound of formula (VII): 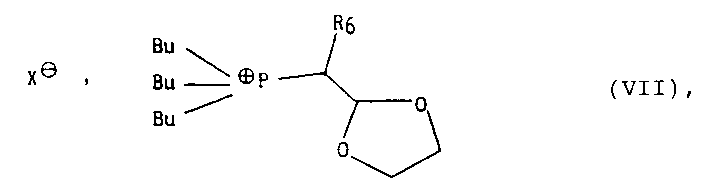 in which X is a halogen atom and R₆ is as defined above,
followed by acid hydrolysis, give the compound of formula (VIII): 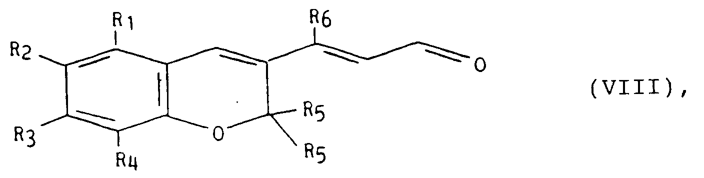 in which R₁, R₂, R₃, R₄, R₅ and R₆ are as defined above,
which compound of formula (VIII), after a second Wittig reaction, under the same conditions, with a compound of formula (VII'): 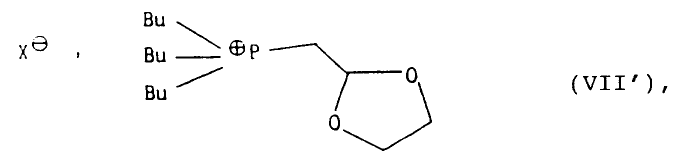 in which X is as defined above,
is converted into the compound of formula (IX): 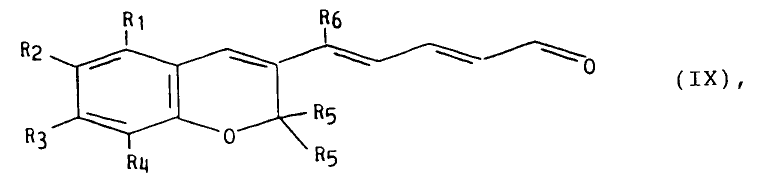 in which the groups R₁ to R₆ are as defined above,
which, finally, is treated with a compound of formula (Xa) or (Xb): 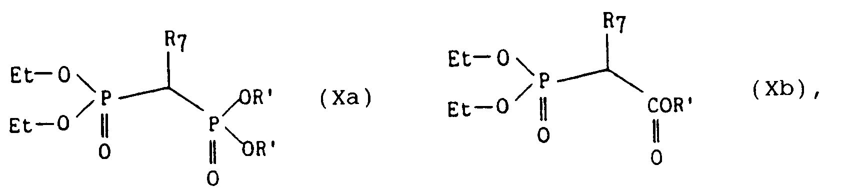 in which R₇ and R' are as defined above,
to give the compounds of formulae (Ia) and (Ib), respectively: 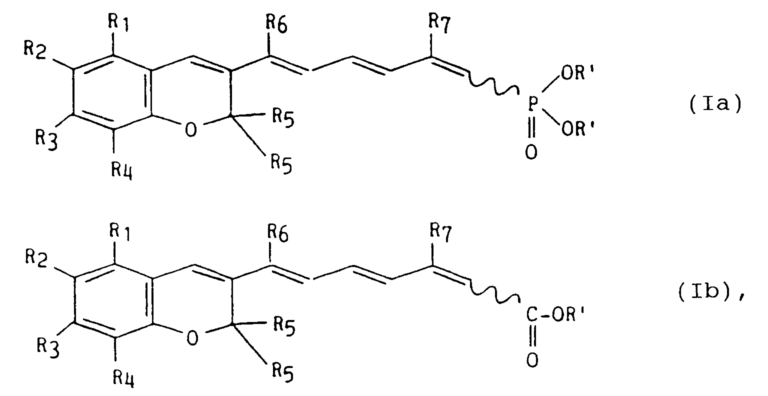 in which the groups R₁ to R₇ and R' are as defined above,
which compound of formula (Ia) may be treated in a basic or acidic medium to obtain the compound of formula (Ic): 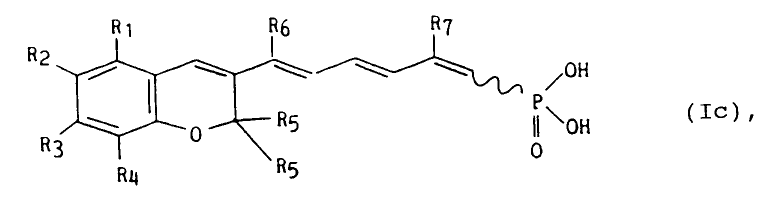 in which the groups R₁ to R₇ are as defined above,
which compound of formula (Ib) may be hydrolysed to form the compound of formula (Id): 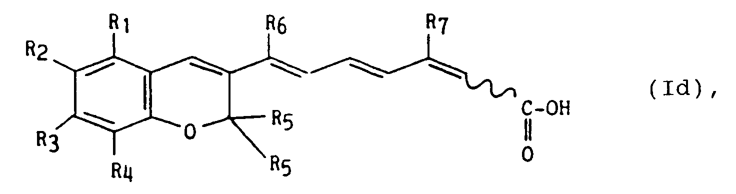 in which the groups R₁ to R₇ are as defined above,
which, in turn, after conversion into its corresponding acyl chloride and treatment with an amine of formula (XI):
R₁₀-NH₂ (XI),
in which R₁₀ is as defined above,
yields the compound of formula (Ie): 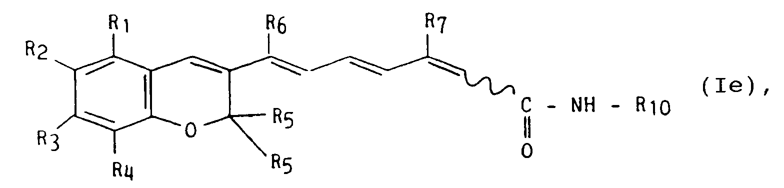 in which the groups R₁ to R₇ and R₁₀ are as defined above,
all the various compounds of formulae (Ia) to (Ie) forming the compounds of the general formula (I) in their entirety, which compounds are purified, where appropriate, by a conventional purification method, and the stereoisomers and the optical isomers of which are separated, if desired, by a conventional separation method, and which are converted, if desired, into their addition salts with a pharmaceutically acceptable acid or base.

9. Pharmaceutical compositions comprising as active ingredient at least one compound according to one of claims 1 to 7 in combination with one or more inert, non-toxic, pharmaceutically acceptable excipients or carriers.

10. Pharmaceutical compositions according to claim 9 comprising at least one active ingredient according to one of claims 1 to 7 having anti-bone resorption properties and properties generating bone tissue, for use in the treatment of osteoporosis and of inflammatory disorders, and in particular in generalised or localised chronic osteopoenic inflammations.

11. Use of the compounds of formula (I'): 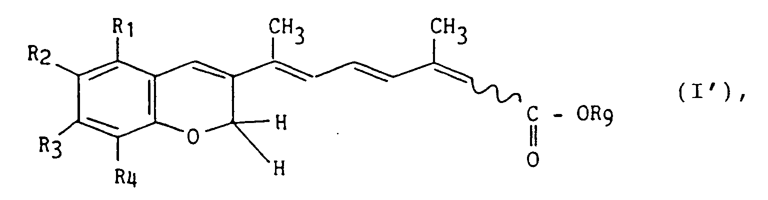 in which:
- R₁, R₂, R₃ and R₄, which are the same or different, are selected from:
- a hydrogen atom,
- a halogen atom,
- a radical R', and
- a radical OR',
- R' being a linear or branched alkyl group containing from 1 to 6 carbon atoms that is optionally substituted by one or more hydroxy groups and/or by one or more halogen atoms,
- R₉ is selected from a hydrogen atom, a linear or branched alkyl group containing from 1 to 6 carbon atoms, and a residue of an amino-sugar, the non-defined bond carrying the radical 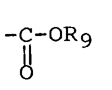 being a single bond conferring a Z- or E-configuration on the double bond supporting it,
and, where applicable, their stereoisomers and addition salts with a pharmaceutically acceptable acid or base, for the preparation of pharmaceutical compositions for use in the treatment of osteoporosis and of inflammatory disorders, and in particular in generalised or localised chronic osteopoenic inflammations.

## Patentansprüche

1. Verbindungen der allgemeinen Formel (I): 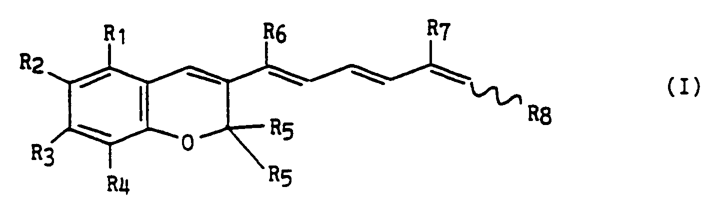 in der:
- R₁, R₂, R₃ und R₄, die gleichartig oder verschieden sind:
- ein Wasserstoffatom,
- ein Halogenatom,
- einen gegebenenfalls substituierten Phenylrest,
- einen Adamantylrest,
- einen gegebenenfalls substituierten heterocyclischen Rest mit 5 oder 6 Atomen ausgewählt aus Furyl-, Pyrrolyl-, Pyrrolidinyl-, Thienyl-, Pyridyl-, Piperidyl-, Indolyl- und Chinolyl-Resten,
- einen Rest R' oder
- einen Rest OR',
- worin R' eine geradkettige oder verzweigte Alkylgruppe mit 1 bis 6 Kohlenstoffatomen darstellt, die gegebenenfalls durch eine oder mehrere Hydroxylgruppen und/oder ein oder mehrere Halogenatome substituiert ist, bedeuten,
- R₅, R₆ und R₇, die gleichartig oder verschieden sind, ausgewählt sind aus Wasserstoffatomen und geradkettigen oder verzweigten Alkylgruppen mit 1 bis 6 Kohlenstoffatomen.
- R₈ ausgewählt ist aus Gruppen C(O)OR₉, P(O)(OR₉)₂ und CONHR₁₀, worin R₉ ein Wasserstoffatom, eine geradkettige oder verzweigte Alkylgruppe mit 1 bis 6 Kohlenstoffatomen oder einen Aminozuckerrest und R₁₀ ein Wasserstoffatom, eine geradkettige oder verzweigte Alkylgruppe mit 1 bis 6 Kohlenstoffatomen oder einen gegebenenfalls substituierten Arylrest darstellen,
wobei die die Gruppe R₈ tragende nicht definierte Bindung eine Einfachbindung darstellt, die der sie tragenden Doppelbindung die Konfiguration E oder Z verleiht, mit der Maßgabe, daß, wenn R₅ Wasserstoff und R₆ und R₇ jeweils eine Methylgruppe und R₈ die Gruppe C(O)OR₉, wie sie oben definiert worden ist, bedeuten, dann mindestens einer der Substituenten R₁, R₂, R₃ oder R₄ von Wasserstoff, Halogen, einer Gruppe R' und einer Gruppe OR' verschieden ist, worin R' die oben angegebenen Bedeutungen besitzt,
deren Stereoisomere sowie deren mögliche Additionssalze mit einer pharmazeutisch annehmbaren Säure oder Base.

2. Verbindung nach Anspruch 1, nämlich (2*E*,4*E*,6*E*)-7-(2,2-Dimethyl-6-methoxy-2*H*-chromen-3-yl)-hepta-2,4,6-triensäure und deren pharmazeutisch annahmbare Additionssalze mit einer Base.

3. Verbindung nach Anspruch 1, nämlich (2*E*,4*E*,6*E*)-7-(6-tert.-Butyl-2*H*-chromen-3-yl)-hepta-2,4,6-triensäure und deren pharmazeutisch annahmbare Additionssalze mit einer Base.

4. Verbindung nach Anspruch 1, nämlich (2*E*,4*E*,6*E*)-7-(6,8-Di-tert.-butyl-2*H*-chromen-3-yl)-hepta-2,4,6-triensäure und deren pharmazeutisch annahmbare Additionssalze mit einer Base.

5. Verbindung nach Anspruch 1, nämlich (2*E*,4*E*,6*E*)-3-Methyl-7-(6-phenyl-2*H*-chromen-3-yl)-octa-2,4,6-triensäure und deren pharmazeutisch annahmbare Additionssalze mit einer Base.

6. Verbindung nach Anspruch 1, nämlich (2*Z*,4*E*,6*E*)-3-Methyl-7-(6-phenyl-2*H*-chromen-3-yl)-octa-2,4,6-triensäureethylester.

7. Verbindung nach Anspruch 1, nämlich (1*E*,3*E*,5*E*)-[2-Methyl-6-(6,8-dimethyl-2*H*-chromen-3-yl)-hepta-1,3,5-trien]-1-phosphonsäurediethylester.

8. Verfahren zur Herstellung der Verbindungen der Formel (I), dadurch gekennzeichnet, daß man die Verbindungen der Formel (II): 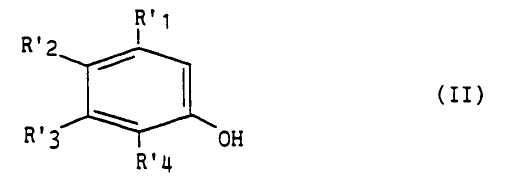 in der R'_{1'} R'_{2'} R'₃ und R'_{4'} die gleichartig oder verschieden sind, Wasserstoffatome, Halogenatome, geradkettige oder verzweigte Alkylgruppen oder Alkoxygruppen mit 1 bis 6 Kohlenstoffatomen, die gegebenenfalls durch eine oder mehrere OH-Gruppen und/oder durch ein oder mehrere Halogenatome substituiert sind, bedeuten, mit Hilfe der Sommelet-Reaktion durch Einwirkung von Paraformaldehyd in Gegenwart von Hexamethylentetramin bei einer Temperatur von 100°C und dann durch Ansäuern,
oder durch Einwirkung eines Metallchlorids in Gegenwart eines Alkylamins mit Trioxymethylen ebenfalls bei 100°C in die Verbindung der Formel (III) umwandelt: 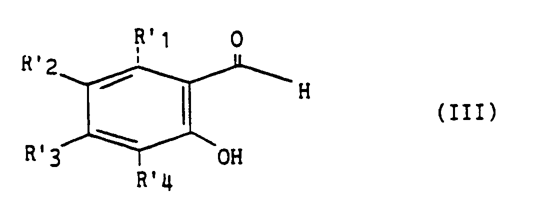 in der R'_{1'} R'_{2'} R'₃ und R'₄ die oben angegebenen Bedeutungen besitzen, welche Verbindung der Formel (III) anschließend durch Einwirkung einer Verbindung der Formel (IV): 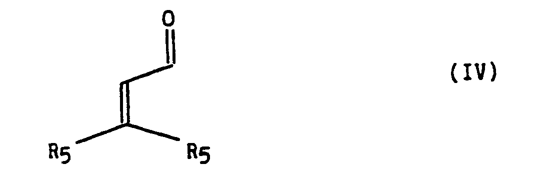 in der R₅ die bezüglich der Formel (I) angegebenen Bedeutungen besitzt, in Gegenwart eines Alkalicarbonats in einem polaren Lösungsmittel am Rückfluß cyclisiert wird zur Bildung der Verbindung der Formel (V): 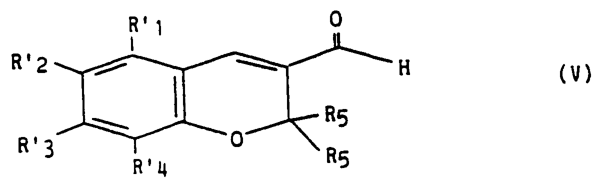 in der R'_{1'} R'_{2'} R'_{3'} R'₄ und R₅ die oben angegebenen Bedeutungen besitzen, welche Verbindung der Formel (V), deren Gruppen R'_{1'} R'_{2'} R'₃ und R'₄ dann, wenn eine dieser Gruppen ein Halogenatom darstellt, durch Reaktion mit einer entsprechenden Tetra-phenyl-, -aryl-, -heterocyclyl- oder heteroaryl-Zinn-Verbindung in Gegenwart eines Katalysators auf Palladiumgrundlage und in Toluol am Rückfluß mit einem Phenylrest, einem substituierten Phenylrest, einem Adamantylrest oder einem heterocyclischen Rest substituiert werden kann, so daß man die Gesamtheit der Verbindungen der Formel (VI) erhält: 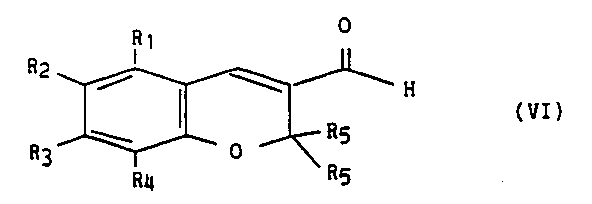 in der R₁, R₂, R₃, R₄ und R₅ die oben angegebenen Bedeutungen besitzen, welche durch Wittig-Reaktion mit Hilfe einer Verbindung der Formel (VII): 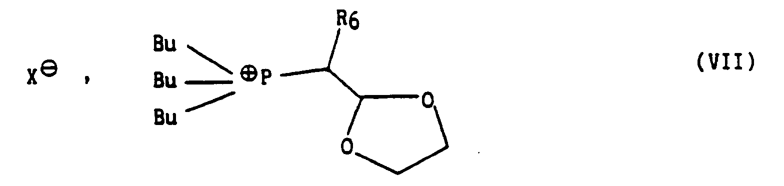 in der X ein Halogenatom darstellt und R₆ die oben angegebenen Bedeutungen besitzt,
und dann durch saure Hydrolyse zu der Verbindung der Formel (VIII) führt: 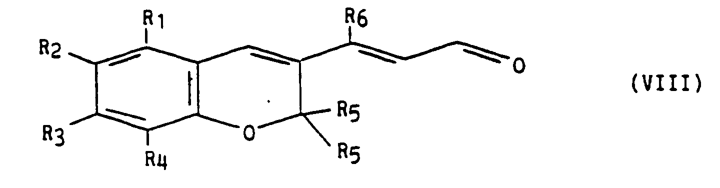 in der R₁, R₂, R₃, R₄, R₅ und R₆ die oben angegebenen Bedeutungen besitzen,
welche Verbindung der Formel (VIII), nachdem sie einer zweiten Wittig-Reaktion unter den gleichen Bedingungen mit einer Verbindung der Formel (VII') unterworfen worden ist: 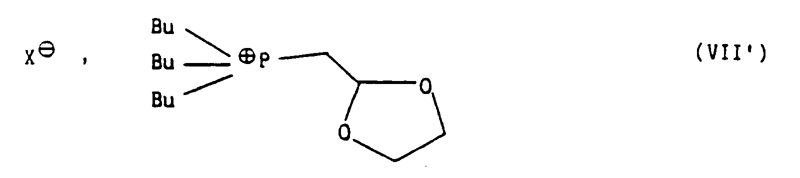 in der X die oben angegebenen Bedeutungen besitzt,
in die Verbindung der Formel (IX) umgewandelt wird: 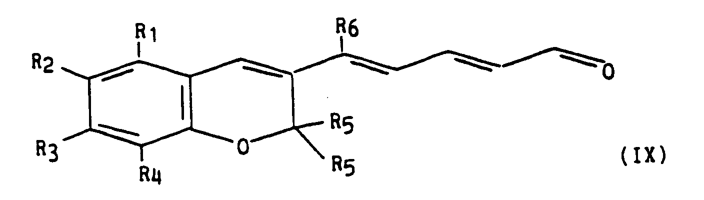 in der die Gruppen R₁ bis R₆ die oben angegebenen Bedeutungen besitzen,
welche schließlich mit einer Verbindung der Formel (Xa) oder (Xb): 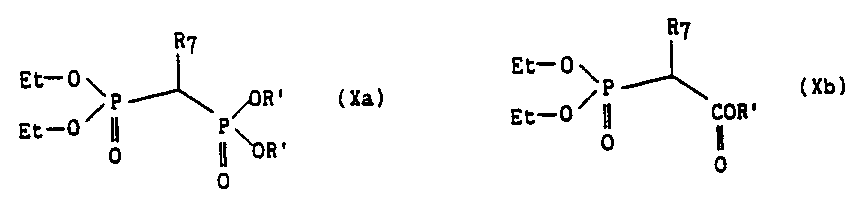 in der R₇ und R' die oben angegebenen Bedeutungen besitzen,
behandelt wird zur Bildung der Verbindungen der Formeln (Ia) bzw. (Ib): 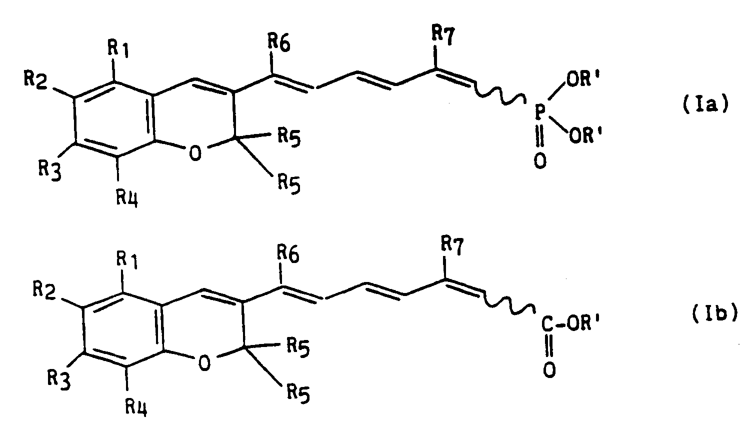 in denen die Gruppen R₁ bis R₇ und R' die oben angegebenen Bedeutungen besitzen.
welche Verbindung der Formel (Ia) durch Behandeln in basischem oder saurem Medium die Bildung der Verbindung der Formel (Ic) ermöglicht; 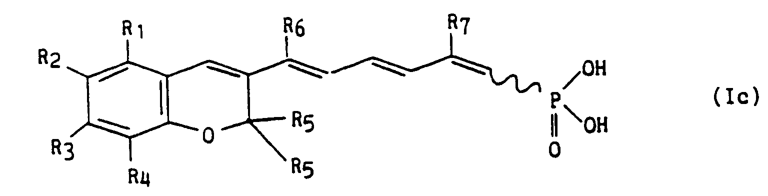 in der die Gruppen R₁ bis R₇ die oben angegebenen Bedeutungen besitzen, welche Verbindung der Formel (Ib) zu der Verbindung der Formel (Id) verseift werden kann: 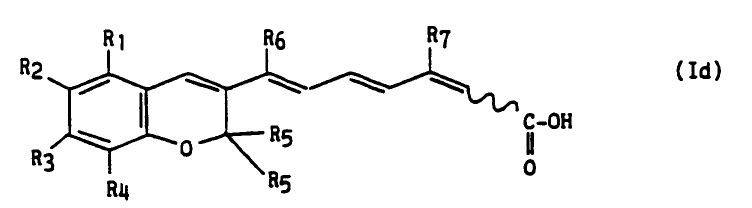 in der die Gruppen R₁ bis R₇ die oben angegebenen Bedeutungen besitzen, welche ihrerseits nach der Umwandlung in das entsprechende Acylchlorid und durch Behandeln mit einem Amin der Formel (XI):
R₁₀ - NH₂ (XI)
in der R₁₀ die oben angegebenen Bedeutungen besitzt, zu der Verbindung der Formel (Ie) führt: 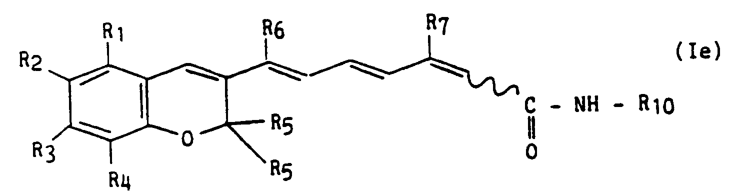 in der die Gruppen R₁ bis R₇ und R₁₀ die oben angegebenen Bedeutungen besitzen,
wobei die Gesamheit der Verbindungen der Formel (Ia) bis (Ie) die Gesamtheit der Verbindungen der allgemeinen Formel (I) bildet, welche man gegebenenfalls mit Hilfe einer klassischen Reinigungsmethode reinigt, gewünschtenfalls mit Hilfe einer klassischen Trennmethode in die Stereoisomeren und die optischen Isomeren auftrennt und welche man gewünschtenfalls mit einer pharmazeutisch annehmbaren Säure oder Base in ihre Additionssalze umwandelt.

9. Pharmazeutische Zubereitungen enthaltend als Wirkstoff mindestens eine Verbindung nach einem der Ansprüche 1 bis 7 in Kombination mit einem oder mehreren inerten, nichttoxischen und pharmazeutisch annehmbaren Trägermaterialien oder Bindemitteln.

10. Pharmazeutische Zubereitungen nach Anspruch 9, enthaltend mindestens einen Wirkstoff nach einem der Ansprüche 1 bis 7 mit Knochen-Antiresorbierwirkung, Knochengewebebildungswirkung und Eignung zur Behandlung von Osteoporose und Entzündungszuständen, insbesondere allgemeinen oder lokalen chronischen osteopenen Entzündungszuständen.

11. Verwendung der Verbindungen der Formel (I'): 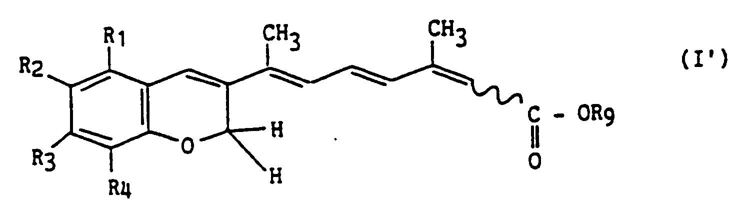 in der:
- R₁, R₂, R₃und R₄, die gleichartig oder verschieden sind, ausgewählt sind aus:
- einem Wasserstoffatom,
- einem Halogenatom,
- einer Gruppe R' und
- einer Gruppe OR',
- worin R' eine geradkettige oder verzweigte Alkylgruppe mit 1 bis 6 Kohlenstoffatomen, die gegebenenfalls durch eine oder mehrere Hydroxylgruppen und/oder ein oder mehrere Halogenatome substituiert ist, darstellt,
- R₉ aus einem Wasserstoffatom, einer geradkettigen oder verzweigten Alkylgruppe mit 1 bis 6 Kohlenstoffatomen oder einem Aminozuckerrest ausgewählt ist, wobei die die Gruppe der Formel 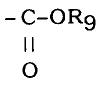 tragende nicht definierte Bindung eine Einfachbindung ist, welche der sie tragenden Doppelbindung die Konfiguration Z oder E verleiht,
sowie deren mögliche Stereoisomere und deren Additionssalze mit einer pharmazeutisch annehmbaren Säure oder Base, zur Herstellung von pharmazeutischenZubereitungen zur Behandlung der Osteoporose und von Entzündungszuständen, insbesondere allgemeinen oder lokalen chronischen osteopenen Entzündungen.
